# EUROPEAN PATENT SPECIFICATION

(11) **EP 3 927 338 B1**
(45) Date of publication and mention of the grant of the patent: **09.10.2024**
(21) Application number: 20710060.3
(22) Date of filing: 24.02.2020
(51) Int. Cl.: A61K 31/4045, A61P 25/24

(54) **COMPOSITIONS COMPRISING 5-METHOXY-N,N-DIMETHYLTRYPTAMINE (5-MEO-DMT) FOR USE IN TREATING MENTAL DISORDERS**
ZUSAMMENSETZUNGEN MIT 5-METHOXY-N,N-DIMETHYLTRYPTAMIN (5-MEO-DMT) ZUR VERWENDUNG BEI DER BEHANDLUNG VON PSYCHISCHEN STÖRUNGEN
COMPOSITIONS COMPRENANT DE LA 5-MÉTHOXY-N,N-DIMÉTHYLTRYPTAMINE (5-MEO-DMT) POUR UNE UTILISATION DANS LE TRAITEMENT DE TROUBLES MENTAUX

(30) Priority: 22.02.2019 EP 19158806
(43) Date of publication of application: 29.12.2021
(73) Proprietor: GH Research Ireland Limited, D02 RY95 Dublin 2 (IE)
(72) Inventor: TERWEY, Theis, 10119 Berlin (DE)
(74) Representative: Breuer, Markus
(86) International application number: PCT/EP2020/054804
(87) International publication number: WO 2020/169851

(56) References cited:
- WO-A1-2005/055996
- WO-A1-2018/195455
- DAVIS ALAN K ET AL: "The epidemiology of 5-methoxy-N, N-dimethyltryptamine (5-MeO-DMT) use: Benefits, consequences, patterns of use, subjective effects, and reasons for consumption", JOURNAL OF PSYCHOPHARMACOLOGY, 30 April 2018 (2018-04-30), pages 779 - 792, XP093006797, Retrieved from the Internet <URL:https://journals.sagepub.com/doi/10.1177/0269881118769063> [retrieved on 20221209]
- BARSUGLIA J P ET AL: "A case report SPECT study and theoretical rationale for the sequential administration of ibogaine and 5-MeO-DMT in the treatment of alcohol use disorder", EVENT-RELATED DYNAMICS OF BRAIN OSCILLATIONS; [PROGRESS IN BRAIN RESEARCH. ISSN 0079-6123], ELSEVIER, AMSTERDAM, NL, vol. 242, 1 January 2018 (2018-01-01), pages 121 - 158, XP009520576, ISSN: 0079-6123, ISBN: 978-0-444-52183-5, DOI: 10.1016/BS.PBR.2018.08.002
- UTHAUG M V ET AL: "A single inhalation of vapor from dried toad secretion containing 5-methoxy-N,N-dimethyltryptamine (5-MeO-DMT) in a naturalistic setting is related to sustained enhancement of satisfaction with life, mindfulness-related capacities, and a decrement of psychopathological symptoms", PSYCHOPHARMACOLOGY, SPRINGER VERLAG, BERLIN, DE, vol. 236, no. 9, 13 April 2019 (2019-04-13), pages 2653 - 2666, XP036863337, ISSN: 0033-3158, [retrieved on 20190413], DOI: 10.1007/S00213-019-05236-W
- CARHART-HARRIS R L ET AL: "Psilocybin with psychological support for treatment-resistant depression: six-month follow-up", PSYCHOPHARMACOLOGY, SPRINGER VERLAG, BERLIN, DE, vol. 235, no. 2, 8 November 2017 (2017-11-08), pages 399 - 408, XP036477494, ISSN: 0033-3158, [retrieved on 20171108], DOI: 10.1007/S00213-017-4771-X
- PORTER R. S. ET AL: "Mood disorders", THE MERCK MANUAL OF DIAGNOSIS AND THERAPY - TWENTIETH EDITION, 1 January 2018 (2018-01-01), pages 1757 - 1766, XP093113475, Retrieved from the Internet <URL:https://www.wiley.com/en-us/The+Merck+Manual+of+Diagnosis+and+Therapy%2C+20th+Edition-p-9780911910421> [retrieved on 20231219]
- EWING C GREER: "Ground to Source-experiencing the Divine within", THE PRACTICAL TRIPPER, 15 April 2017 (2017-04-15), pages 1 - 20, XP093143625, Retrieved from the Internet <URL:https://thepracticaltripper.wordpress.com/2017/04/15/ground-to-source-experiencing-the-divine-within-2/> [retrieved on 20240320]
- ANONYMOUS: "What is depression?", 1 January 2019 (2019-01-01), pages 1 - 6, XP093143634, Retrieved from the Internet <URL:https://web.archive.org/web/20190117034902/https://www.psychiatry.org/patients-families/depression/what-is-depression> [retrieved on 20240320]
- JOSEPH BARSUGLIA ET AL: "Intensity of Mystical Experiences Occasioned by 5-MeO-DMT and Comparison With a Prior Psilocybin Study", FRONTIERS IN PSYCHOLOGY, vol. 9, 6 December 2018 (2018-12-06), XP055695516, DOI: 10.3389/fpsyg.2018.02459
- ALAN K DAVIS ET AL: "The epidemiology of 5-methoxy- N, N -dimethyltryptamine (5-MeO-DMT) use: Benefits, consequences, patterns of use, subjective effects, and reasons for consumption", JOURNAL OF PSYCHOPHARMACOLOGY., vol. 32, no. 7, 30 April 2018 (2018-04-30), GB, pages 779 - 792, XP055695664, ISSN: 0269-8811, DOI: 10.1177/0269881118769063
- ALAN K. DAVIS ET AL: "5-methoxy- N,N -dimethyltryptamine (5-MeO-DMT) used in a naturalistic group setting is associated with unintended improvements in depression and anxiety", AMERICAN JOURNAL OF DRUG AND ALCOHOL ABUSE, vol. 45, no. 2, 4 March 2019 (2019-03-04), US, pages 161 - 169, XP055695498, ISSN: 0095-2990, DOI: 10.1080/00952990.2018.1545024

## Description

### Technical Field

The present invention is directed to improved methods for the treatment of mental disorders, namely major depressive disorder, comprising administering to a patient in need thereof a therapeutically effective amount of 5-methoxy-N,N-dimethyltryptamine (5-MeO-DMT). 5-MeO-DMT is administered via the intravenous, intramuscular or subcutaneous route.

### Background of the Invention

Hallucinogens are chemical compounds, some naturally occurring, some synthetic, which are defined by their ability to induce in humans after consumption sensory distortions, such as changes in auditory and visual perception, as well as distortions of mood and cognition. The term hallucinogen encompasses a rather broad group of psychoactive molecules with different modes of action. Some mental disorders have been suggested as in principle being amenable for treatment with psychoactive molecules.

Document BARSUGLIA J P ET AL: "A case report SPECT study and theoretical rationale for the sequential administration of ibogaine and 5-MeO-DMT in the treatment of alcohol use disorder", EVENT-RELATED DYNAMICS OF BRAIN OSCILLATIONS; [PROGRESS IN BRAIN RESEARCH. ISSN 0079-6123], ELSEVIER, AMSTERDAM, NL, vol. 242, 1 January 2018 (2018-01-01), pages 121-158, presents the case of a 31-year-old male military veteran with moderate alcohol use disorder who sought treatment that utilized a sequential protocol with ibogaine hydrochloride (1550mg, 17.9mg/kg) on day 1, followed by vaporized 5-MeO-DMT (bufotoxin source 50 mg, estimated 5-MeO-DMT content, 5-mg) on day 3.

Document CARHART-HARRIS R L ET AL: "Psilocybin with psychological support for treatment-resistant depression: six-month follow-up", PSYCHOPHARMACOLOGY, SPRINGER VERLAG, BERLIN, DE, vol. 235, no. 2, 8 November 2017 (2017-11-08), pages 399-408, discloses the use of psilocybin, a phosphate derivative of DMT, for treating treatment resistant depression in patients, at two oral doses of psilocybin (10 and 25 mg), 7 days apart, together with psychological support.

### Summary of Invention

The invention is defined by the claims. Any subject-matter falling outside the scope of the claims is provided for information purposes only. The references to methods of treatment in the detailed description of the invention in this description are to be interpreted as references to the compounds, pharmaceutical compositions and medicaments of the present invention for use in a method for treatment of the human (or animal) body by therapy.

The present invention relates to 5-Methoxy-N,N-dimethyltryptamine (5-MeO-DMT) or a pharmaceutically acceptable salt thereof for use in treating major depressive disorder in a patient who is diagnosed with major depressive disorder by a licensed professional in accordance with accepted medical practice, wherein the 5-MeO-DMT is administered via the intravenous, intramuscular or subcutaneous route.

The disorder may be diagnosed in accordance with the Diagnostic and Statistical Manual of Mental Disorders - Fifth Edition (DSM-5) published by the American Psychiatric Association. For instance, the patient may suffer from moderate or severe major depressive disorder as indicated by a Montgomery-Åsberg Depression Rating Scale (MADRS) score of 20 or more or by a Hamilton Depression Rating Scale (HAM-D) score of 17 or more. It is further considered that the patient may suffers from severe major depressive disorder as indicated by a Montgomery-Åsberg Depression Rating Scale (MADRS) score of 35 or more or by a Hamilton Depression Rating Scale (HAM-D) score of 25 or more. The patient may be diagnosed with a treatment-resistant form of major depressive disorder.

Further, the patient may suffer from suicidal ideation, in particular from suicidal ideation with intent to act. The patient may even be at imminent risk for suicide.

In the context of the present invention, 5-MeO-DMT or a pharmaceutically acceptable salt thereof is administered via intravenous, intramuscular or subcutaneous administration.

5-MeO-DMT or a pharmaceutically acceptable salt thereof may be administered at a dose or in a dosage regimen that causes the patient to experience a peak psychedelic experience. A dosage of about 1 mg to about 10 mg 5-MeO-DMT or an equimolar amount of a pharmaceutically acceptable salt may be administered.

5-MeO-DMT or a pharmaceutically acceptable salt may be administered in a first dosage amount for a first administration; and then be administered in zero to six subsequent administrations; wherein each subsequent administration uses a dosage amount higher than the previous administration unless the patient experiences a peak psychedelic experience.

The interval between two administrations may not be less than 1 hour and not more than 24 hours and may preferably be about 2 to 4 hours.

The occurrence of a peak psychedelic experience can be identified through achievement of at least 60% of the maximum possible score in each of the four subscales (mystical, positive mood, transcendence of time and space, and ineffability) of the 30-item revised Mystical Experience Questionnaire (MEQ30) or is identified through achievement of at least 60% of the maximum possible score of the Oceanic Boundlessness (OBN) dimension of the Altered States of Consciousness (ASC) questionnaire or preferably is identified through achievement of a Peak Psychedelic Experience Questionnaire (PPEQ) Total Score of at least 75.

Treatment as indicated above with 5-MeO-DMT or a pharmaceutically acceptable salt thereof leads to a clinical response. The response may be assessed by at least a score of "much improved" in the Clinical Global Impression - Improvement (CGI-I) score or the Patient Global Impression - Improvement (PGI-I) score, which improvement preferably occurs not later than about 2 hours after the last administration of 5-MeO-DMT or a pharmaceutically acceptable salt thereof.

The clinical response, as assessed by at least a score of "much improved" in the CGI-I score or the PGI-I score, preferably persists until at least 6 days, more preferably at least 14 days and in particular at least 28 days, after the last administration of 5-MeO-DMT or a pharmaceutically acceptable salt thereof.

The clinical response may also be assessed by at least 50% improvement of the MADRS or HAM-D score, compared to the respective score prior to administration of 5-MeO-DMT. This response preferably occurs not later than about 2 hours after the last administration of 5-MeO-DMT or a pharmaceutically acceptable salt thereof. Further, a remission of depressive symptoms, as assessed by a MADRS score equal to or less than 10, or a HAM-D score equal to or less than 7, preferably occurs not later than about 2 hours after the last administration of 5-MeO-DMT or a pharmaceutically acceptable salt thereof.

The clinical response, as assessed by at least 50% improvement of the MADRS or HAM-D score, compared to the respective score prior to treatment, preferably persists until at least 6 days, more preferably until at least 14 days, in particular until at least 28 days, after the last administration of 5-MeO-DMT or a pharmaceutically acceptable salt thereof.

Preferably, there is a clinical response, as assessed by at least 75% improvement of the MADRS or HAM-D score, compared to the respective score prior to treatment, on day 7, more preferably also on day 14 and in particular also on day 28, after the last administration of 5-MeO-DMT or a pharmaceutically acceptable salt thereof. Further preferably, the patient is in remission of depressive symptoms, as assessed by a MADRS score equal to or less than 10, or a HAM-D score equal to or less than 7, on day 7 preferably also on day 14, in particular also on day 28, after the last administration of 5-MeO-DMT or a pharmaceutically acceptable salt thereof.

### Detailed Description of the Invention

Hallucinogens include, e.g., the cannabinoid tetrahydrocannabinol (THC) which acts on cannabinoid receptors, the entactogen 3,4-methylenedioxymethamphetamine (MDMA, "Ecstasy") which acts on trace amine-associated receptor 1 (TAAR1) and vesicular monoamine transporter 2 (VMAT2), and the dissociative anesthetic ketamine which acts as a N-methyl-D-aspartate (NMDA) receptor antagonist.

A further group of hallucinogens entails the compounds which bind to the 5-hydroxytryptamine (5-HT) receptors, or serotonin receptors (described are 7 families 5-HT1 to 5-HT7 with 14 subtypes), such as lysergic acid diethylamide (LSD), psilocybin, and N,N-dimethyltryptamine (DMT). This latter group of serotonergic agents is often referred to as "classical hallucinogens" or "psychedelics", which emphasizes their predominant ability to induce qualitatively altered states of consciousness such as euphoria, trance, transcendence of time and space, spiritual experiences, dissolution of self-boundaries, or even near-death experiences, while other effects such as sedation, narcosis, or excessive stimulation are only minimal.

Chemically, serotonergic psychedelics are either phenylalkylamines or indoleamines, with the indoleamine class being divided into two subsets, ergolines and tryptamines, the latter being derived from tryptamine having the following formula:

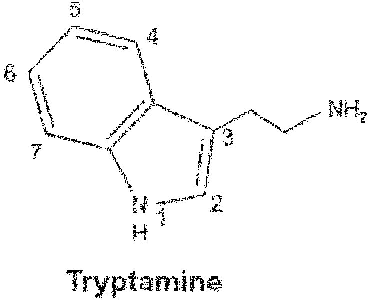

The various serotonergic psychedelics have different binding affinity and activation potency for various serotonin receptors, particularly 5-HT1A, 5-HT2A, and 5-HT2C, and their activity may also be modulated by interaction with other targets such as monoamine transporters and trace amine-associated receptors.

Naturally occurring psychedelics, such as the tryptamine DMT, which is contained in the South American shrub *Psychotria viridis,* or the tryptamine psilocybin, which is contained in over 200 mushroom species, or the phenylalkylamine mescaline, which is contained in the Peyote cactus of the American Southwest and Northern Mexico, have been used for centuries by indigenous cultures in ritual or sociocultural contexts and in the context of religious sacraments. While an unspecific "healing" potential had been ascribed to the use of naturally occurring psychedelics in those settings, more scientific investigations into their potential therapeutic application for defined disease entities had not been pursued until after the discovery of the synthetic ergoline LSD in 1943.

Those early clinical experiences of the 1950s and 1960s were methodologically still relatively weak, but encouraging data was e.g. reported from a treatment program of alcoholism, where patients who received LSD together with psychotherapy had higher rates of abstinence or improvement than patients who received psychotherapy alone (Jensen SE, QJ Stud Alcohol. 1962; 23:315-20), or from observations in patients with personality trait disturbance or anxiety where administration of LSD as an aid to psychotherapy yielded an outcome of "much improved" or "improved" in over 90% of patients (MacLean JR et al., QJ Stud Alcohol. 1961; 22:34-45), or from treatment of patients suffering from terminal state of cancer, where about two-thirds of patients who received LSD treatment improved in varying degrees (Pahnke WN et al., Psychedelic drugs. Proceedings of a Hahnemann Medical College and Hospital Symposium. Editors: Richard E. Hicks, Paul Jay Fink, Van Buren O. Hammett. Grune & Stratton, New York/London 1969, pp.33-42).

With the emerging knowledge about the serotonin system and its role in brain function, researchers more and more specified the molecular activity of the psychedelic drugs. However, how that activity translated into their observed therapeutic effects in mental disorders was less clear. Two main concepts were proposed: The first concept was coined "psycholytic therapy" and it emphasized the ability of psychedelics given at low doses to facilitate the loosening of psychological defensive mechanisms, which in combination with psychotherapy allows a deep introspective insight and the revival of traumata and their subsequent catharsis. The basic mechanism considered in the psycholytic approach was therefore the activation and deepening of the concomitant psychotherapeutic process, and it required multiple drug and therapy sessions. The second concept was coined as "psychedelic therapy" and it emphasized the ability of psychedelics given at high single doses to induce so called "peak psychedelic experiences". Peak experiences are predominantly characterized by the loss of judgment to time and space and the dissolution of ego boundaries, which often culminates in the experience of a blissful state and feelings of being a whole and harmonious existence in the cosmic unity. The basic mechanism considered in the psychedelic approach was therefore to produce a unique, overwhelming experience with an intuitive perception of psychological integration and harmony and subsequent self-improvements and enhanced joy in living and a sense of inner peace.

While scientific research around the use of psychedelics for the treatment of mental disorders blossomed in the 1960s, there was also a rapidly growing recreational use of these substances, and soon psychedelics were depicted in the media as highly dangerous drugs of abuse. A perceived danger to the social order led to the passage of the United States Controlled Substances Act of 1970, under which LSD and other psychedelics were placed into the most restrictive category Schedule 1, which contains drugs deemed to have no medical use and a high potential for abuse. Very little progress was made regarding possible therapeutic uses of psychedelic drugs for the next 30 years.

Only recently has the interest in the field of psychedelic therapy resurged. For example, in a randomized, double-blind, cross-over trial comparing a very low placebo-like single dose of psilocybin with a high single dose in 51 cancer patients with anxiety and/or mood symptoms, the high dose produced large decreases in clinician- and self-rated measures of depression, anxiety or mood disturbances and increases in measures of quality of life at five weeks after treatment, and these effects were sustained at 6-months (Griffiths RR et al., J Psychopharmacol. 2016; 30(12):1181-1197). In another randomized, double-blind, cross-over trial of a single dose of psilocybin with niacin as comparator in 29 cancer patients with anxiety and depression psilocybin produced immediate and substantial improvements in anxiety and depression and led to decreases in cancer-related demoralization and hopelessness and increased quality of life, with enduring effects at the 6.5-month follow-up (Ross Set al., J Psychopharmacol. 2016; 30(12):1165-1180). Further positive experiences with psilocybin were generated in an open-label, single arm feasibility study where 20 patients with treatment-resistant depression (as specifically defined in the study) received a low dose and a high dose of psilocybin seven days apart, and in this trial depressive symptoms were significantly reduced from baseline at one and five weeks after treatment (Carhart-Harris RL et al., Lancet Psychiatry. 2016; 3(7):619-27; Carhart-Harris RL et al., Psychopharmacology (Berl). 2018; 235(2):399-408). Psilocybin has also shown promise in the treatment of obsessive-compulsive disorder (Moreno FA et al., J Clin Psychiatry. 2006; 67(11):1735-40) and alcohol (Bogenschutz MP et al., J Psychopharmacol. 2015; 29(3):289-99) and tobacco dependency (Johnson MW et al., J Psychopharmacol. 2014; 28(11):983-92; Johnson MW et al., Am J Drug Alcohol Abuse. 2017; 43(1):55-60). Further, for the serotonergic psychedelic LSD, a double-blind, randomized, active placebo-controlled pilot study in 12 patients with anxiety associated with life-threatening diseases reported significant effects on anxiety measures at two months follow-up (Gasser P et al., J Nerv Ment Dis. 2014; 202(7):513-20). Also, for the shamanic brew Ayahuasca, which contains the psychoactive agent DMT together with the monoamine oxidase (MAO) inhibitors harmine, harmaline and tetrahydroharmine, a randomized, double-blind, placebo-controlled trial of a single dosing session in 35 patients with treatment-resistant major depression (as specifically defined in the trial) showed a rapid antidepressant effect compared to placebo (Palhano-Fontes F et al., bioRxiv 103531. 2017; doi: https://doi.org/10.1101/103531).

The inventor considers that those results show that administration of certain serotonergic psychedelics may be a promising approach for the treatment of various mental disorders. However, at this point efficacy and safety studies complying with regulatory standards have yet to be conducted and no psychedelic drug has been approved by any regulatory agency. Further, it is evident from the currently available data that none of the tested drugs will achieve remissions in all patients, that some patients may lose response after achieving a remission, and that the tested drugs still suffer from relevant side effects. Also, the acute psychedelic effects after oral dosing of psychedelics currently proposed for therapeutic use have a duration of several hours, which is inconvenient for the patient and the provider and which poses significant limitations in their practical use. For example, in the study of psilocybin in treatment-resistant depression (as specifically defined in the study) published by Carhart-Harris et al. 9 of 20 patients had a response at week 5, but 3 relapsed again until month 6, with frequently reported side effects of anxiety, headache and nausea, and monitoring requirements for at least 7 hours after drug administration, based on a duration of the acute psychedelic effects of psilocybin of approximately 5 to 6 hours (Carhart-Harris RL et al., Lancet Psychiatry. 2016; 3(7):619-27; Carhart-Harris RL et al, Psychopharmacology (Berl). 2018; 235(2):399-408). Additionally, not only a shorter duration of psychedelic effects, but also a more rapid onset of clinical response compared with currently available treatments and currently studied psychedelics would be beneficial. Further, while some mental disorders have been suggested as in principle being amenable for treatment with psychedelics, specific disease entities and specific subgroups of disease entities remain to be identified. Therefore, an aim of the current invention is to provide a compound for improved psychoactive therapies as well as dosing regimens and administration routes for such therapies which are more effective (i.e., (a) larger percentage of patients experiencing a clinical response, b) a larger average clinical response, c) an earlier onset of the clinical response, d) a more durable clinical response) than previously described therapies. A further aim of the current invention is to provide a compound for improved psychoactive therapies as well as dosing regimens and administration routes for such therapies which have a better safety profile and/or are better tolerated than previously described therapies. Another aim of the current invention is to provide a compound for improved psychoactive therapies as well as dosing regimens and administration routes for such therapies which are more convenient. Another aim of the current invention is to provide a compound for improved psychoactive therapies as well as dosing regimens and administration routes for such therapies which are associated with higher rates of patient compliance (including higher rates of treatment initiation) than previously described therapies. A still further aim of the current invention is to identify specific disease entities and specific subgroups of disease entities which benefit from such improved psychoactive therapies.

The currently available treatments for mental disorders, in particular for major depressive disorder, persistent depressive disorder, anxiety disorder, posttraumatic stress disorder, body dysmorphic disorder, obsessive-compulsive disorder, eating disorder and psychoactive substance abuse are unsatisfactory because of the often limited or not durable treatment response, the late onset of response, side effects which limit the long-term drug administration, and inconvenient dosing regimens which often limit compliance of the patient.

Recently published clinical studies which have used serotonergic psychedelic drugs such as LSD, psilocybin and DMT (using the shamanic brew Ayahuasca) in some of those mental disorders suggest that those compounds could provide an alternative to the currently available treatments. However, even for those drugs still not all patients will respond, some patients will lose response over time, and specific side effects will occur. Further, the acute psychoactive effects of the so far tested compounds based on current dosing regimens and administration routes persist for several hours after administration, which is a significant implementation and convenience problem. Also, the onset of clinical response with those compounds is not rapid enough, e.g. for treatment of very symptomatic patients or for patients with active suicidal ideation with intent to act, including such patients who are at imminent risk for suicide. Finally, while some indications have been shown to be in principle amenable for treatment with those serotonergic psychedelic agents, specific disease entities and specific subgroups of disease entities remain to be identified.

The technical problem to be solved by the present invention is therefore in a broad sense to provide an improved psychoactive therapy based on the application of a serotonergic psychedelic in a patient with a mental disorder. The technical problem further encompasses the identification of specific mental disorders and subgroups of mental disorders amenable for treatment with such improved therapy.

Although it is believed that most serotonergic psychedelics mediate their psychoactive effects primarily via 5-HT2A receptors, other receptors also play a role and the complete pharmacology is rather complex. Because there is furthermore no clear correlation between the various mental conditions and specific 5-HT receptor systems, it is not easily predictable which specific receptor affinity profile of a psychedelic will provide the optimal therapeutic effects.

The inventor has recognized that the occurrence of a peak psychedelic experience during the acute phase after administration of a specific psychedelic is driving its therapeutic benefit, either in a causal relationship or at least as a surrogate behavioral marker for the underlying unknown therapeutic mechanism.

The inventor considers that the relevance of the type and intensity of psychedelic experiences is e.g. supported by the study on depression and anxiety in patients with life-threatening cancer where Griffiths et al. reported that the immediate post-session mystical experience score after administration of psilocybin showed a significant association with various therapeutic outcome measures 5 weeks later (Griffiths RR et al., J Psychopharmacol. 2016; 30(12):1181-1197). Also, in the comparable study in cancer patients with anxiety and depression reported by Ross et al. it was found that intensity of the subjective mystical experience during the drug exposure significantly mediated (e.g. suggestive of causality) the clinical benefit (Ross S et al., J Psychopharmacol. 2016; 30(12):1165-1180). In the study of psilocybin in treatment-resistant depression (as specifically defined in the study), it was found that Oceanic Boundlessness (OBN) (sharing features with mystical-type experiences, e.g. experiences of unity and blissful state) was significantly more predictive of reduced depressive symptoms than psilocybin's more generic visual and auditory perceptual effects (Roseman Let al., Front Pharmacol. 2018; 8:974). Further, in the study of psilocybin in alcohol dependence by Bogenschutz et al. it was found that the intensity of effects in the first psilocybin session strongly predicted change in drinking during weeks 5-8 (Bogenschutz MP et al., J Psychopharmacol. 2015; 29(3):289-99). Also, in the study on tobacco dependence by Johnson et al. it was found that those who were smoke-free at six months scored significantly higher on a measure of psilocybin-occasioned mystical experience compared to those who had relapsed (Johnson MW et al., J Psychopharmacol. 2014; 28(11):983-92; Garcia-Romeu A, Curr Drug Abuse Rev. 2014;7(3):157-64). Similar observations had already been made in the early days of psychedelic research, where e.g. in patients with alcohol dependence who had received therapy with LSD it was reported that "50 per cent of these people are changed [that is, they stop drinking or are much improved] ... As a general rule ... those who have not had the transcendental experience are not changed; they continue to drink. However, the large proportion of those who have had it are changed" (cited in Unger SM, Psychiatry. 1963; 26:111-25). In fact, those early experiences had spurred the previously discussed concept of "psychedelic therapy".

The inventor believes that the prominent role of the type and intensity of the acute psychedelic experience for long-term clinical improvement in such a broad range of mental conditions can be explained by recent observations regarding human brain functional connectivity (FC) via so called resting-state networks (RSNs). Those RSNs have been shown to be responsible for various aspects of complex cognitive function, and it has been found that these connectivity networks can be disturbed in mental disorders, in particular major depressive disorder, persistent depressive disorder, anxiety disorder, posttraumatic stress disorder, body dysmorphic disorder, obsessive-compulsive disorder, eating disorder and psychoactive substance abuse, and also in patients with suicidal ideation which can be comorbid with those diseases.

The inventor believes that disruption of the normal hierarchical architecture of RSNs is a final common pathway of those diseases, which may also explain why many of the involved diseases can occur in the same patient at the same time. The inventor considers that the further showings that a) administration of psychedelics such as psilocybin, LSD and DMT can lead to decreased connectivity within specific RSNs (Carhart-Harris RL et al., Sci Rep. 2017; 7(1):13187; Palhano-Fontes F et al., PLoS One. 2015; 10(2):e0118143; Carhart-Harris RL et al., Proc Natl Acad Sci USA. 2016; 113(17):4853-8), that b) this decreased connectivity correlates with ratings of peak experiences (Carhart-Harris RL et al., Sci Rep. 2017;7(1):13187; Carhart-Harris RL et al., Proc Natl Acad Sci USA. 2016; 113(17):4853-8), and that c) after the peak experience an increased reorganization (or normalization) of specific RSN activity can be observed (Carhart-Harris RL et al., Sci Rep. 2017; 7(1):13187), which d) correlates with treatment response (Carhart-Harris RL et al., Sci Rep. 2017; 7(1):13187), explains the observations that the type and intensity of the acute psychedelic experience is correlated with therapeutic outcome.

The inventor has recognized that the occurrence of a peak experience is an important mechanism or at the least a surrogate behavioral marker for the underlying mechanism for the therapeutic efficacy of a psychedelic drug. Therefore, the inventor has recognized that achieving peak experiences more rapidly, in a larger proportion of patients and with better reproducibility in an individual patient, compared with previously tested psychedelic agents and their dosing regimens and administration routes, will lead to a better therapeutic profile.

The inventor has identified 5-MeO-DMT (see formula below) as a serotonergic psychedelic with larger propensity to induce peak experiences than the serotonergic psychedelics previously studied for the treatment of mental disorders, and the inventor has also recognized that peak experiences under 5-MeO-DMT more often involve the dissolution of ego boundaries and experiences of a blissful state and/or unity. The inventor has also recognized that 5-MeO-DMT can induce peak experiences more rapidly than the serotonergic psychedelics previously studied for the treatment of mental disorders, and that the duration of the psychedelic experience is shorter. The inventor considers that those characteristics of 5-MeO-DMT are associated with an improved therapeutic profile and can be explained by specific alterations of RSN activity under 5-MeO-DMT treatment.

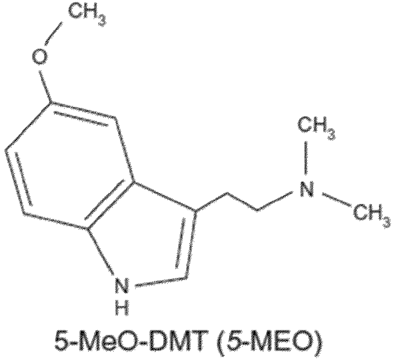

The invention relates to 5-methoxy-N,N-dimethyltryptamine (5-MeO-DMT) or a pharmaceutically acceptable salt thereof for use in treating major depressive disorder in a patient who is diagnosed with major depressive disorder (MDD) by a licensed professional in accordance with accepted medical practice, wherein the 5-MeO-DMT is administered via the intravenous, intramuscular or subcutaneous route.

In one aspect, the disclosure relates to the use of therapeutically effective amounts of 5-MeO-DMT in the treatment of mental disorders, in particular major depressive disorder, persistent depressive disorder, anxiety disorder, posttraumatic stress disorder, body dysmorphic disorder, obsessive-compulsive disorder, eating disorder and psychoactive substance abuse. The use of therapeutically effective amounts of 5-MeO-DMT in the treatment of such disorders includes the use of therapeutically effective amounts of 5-MeO-DMT in patients with such a disorder and suicidal ideation. It in particular includes the use of therapeutically effective amounts of 5-MeO-DMT in patients with a treatment-resistant form of such a disorder, including the use in patients with suicidal ideation. All those conditions are known to be associated with disturbed activity of RSNs, and the inventor has recognized that this makes them amenable for treatment with 5-MeO-DMT.

In another aspect, the disclosure relates to the use of therapeutically effective amounts of 5-MeO-DMT in the treatment of mental disorders, in particular major depressive disorder, persistent depressive disorder, anxiety disorder, posttraumatic stress disorder, body dysmorphic disorder, obsessive-compulsive disorder, eating disorder and psychoactive substance abuse, whereby a clinical response is achieved rapidly after administration of 5-MeO-DMT.

In another aspect, the disclosure relates to the use of therapeutically effective amounts of 5-MeO-DMT in the treatment of mental disorders, in particular major depressive disorder, persistent depressive disorder, anxiety disorder, posttraumatic stress disorder, body dysmorphic disorder, obsessive-compulsive disorder, eating disorder and psychoactive substance abuse, whereby a clinical response persists for extended periods of time after administration of 5-MeO-DMT.

In another aspect, the disclosure relates to novel dosing regimens and administration routes of 5-MeO-DMT for use in the treatment of mental disorders, in particular major depressive disorder, persistent depressive disorder, anxiety disorder, posttraumatic stress disorder, body dysmorphic disorder, obsessive-compulsive disorder, eating disorder and psychoactive substance abuse, which novel dosing regimen and administration routes allow achievement of peak experiences in a large proportion of patients and with good reproducibility within the same patient. Besides allowing for a high rate of peak experiences and consequently improved therapeutic effects those novel dosing regimens and administration routes at the same time reduce the risk of overdosing which improves the safety profile. Such improved dosing regimens rely on the application of increasing dosages of 5-MeO-DMT to the same patient on subsequent administrations on the same day or on directly subsequent days. Such improved administration routes rely on the intravenous, intramuscular or subcutaneous administration of 5-MeO-DMT.

Via each of those aspects and in particular by a combination of those aspects the invention solves the problems of 1) providing an improved psychoactive therapy based on the application of a psychedelic in a patient with a mental disorder and 2) identifying specific mental disorders and specific subgroups of mental disorders amenable for treatment with such improved therapy.

The fact that 5-MeO-DMT has improved therapeutic effects despite its short duration of acute psychedelic effects (5 to 20 minutes after intravenous injection compared with several hours for e.g. oral psilocybin and oral LSD) is surprising, but in fact this short duration of action, and the absence of relevant tolerance (i.e. the absence of diminished or no psychedelic effects after re-administration), is a basis for enabling the novel dosing regimen with frequent re-administrations (such as more than once daily, or daily), which are designed to increase the rate of occurrence of peak experiences, thereby increasing the therapeutic benefit. Such repeat administrations within short time also allow an intraindividual dose-optimization which reduces the risk of overdosing, which may otherwise lead to somatic side effects, such as the serotonin syndrome, negative psychic reactions, such as flashbacks of the experience at later timepoints, or to less meaningful psychedelic experiences with few or no memories of the altered state (so-called "white-outs"). Further, starting with a low dose allows familiarization of the patient with the psychedelic experience in general, and allows preparation for the more intense symptoms to occur at the higher doses, which will positively influence the experience at those higher doses. Also, the prospect of being able to initiate treatment with a low dose will increase patient acceptance of the therapeutic approach and improve overall compliance rates on the patient population level.

Frequent re-administrations of a serotonergic psychedelic with the aim to increase the rate and tailor the reproducibility of peak experiences and to improve the therapeutic effect, reduce the side effects and improve the compliance rates have not been contemplated in the prior art, and in fact they may not be possible with the currently tested administration regimens for other psychedelics, due to the late onset and long duration of psychedelic effects observed with their current dosing regimens and administration routes and due to the rapid development of tolerance (i.e. diminished or no psychedelic effects after re-administration) which can last for several days. The hitherto described dosing regimens for psychedelic drugs used in the therapeutic context either contemplated only a single administration or repeat administrations only after several days, and they only contemplated oral administration. Hence, those currently applied dosing regimens and administration routes do not allow peak experiences to be reliably achieved and they do not offer the additional benefits as described above.

The intravenous, intramuscular or subcutaneous administration of 5-MeO-DMT allows for high bioavailability and low variability in bioavailability between patients and between re-administrations in the same patient, and it therefore allows for a high rate and a high reproducibility of peak experiences. Also, intravenous, intramuscular or subcutaneous administration will be considered at least by some patients as more convenient and better tolerable as compared to administration via inhalation or intranasal insufflation, and intravenous, intramuscular or subcutaneous administration will be preferred by at least some physicians for ease of use. Further, intravenous, intramuscular or subcutaneous administration avoids exposure of the physician or caregiver to 5-MeO-DMT, which could occur when applied via the inhalation route.

5-MeO-DMT is a naturally occurring serotonergic psychedelic tryptamine which acts as a 5-HT1A and 5-HT2A receptor agonist. 5-MeO-DMT was first isolated from the bark of *Dictyoloma incanescens*, but it is also contained in other plants, and it has been identified as the major active ingredient in the venom of *Bufo alvarius* toads. In addition, 5-MeO-DMT is synthesized in human pineal and retina, and it has been found in human body fluids including urine, blood, and cerebrospinal fluid. 5-MeO-DMT is mainly inactivated through a deamination pathway mediated by monoamine oxidase A, and it is O-demethylated by cytochrome P450 2D6 (CYP2D6) enzyme to produce an active metabolite, bufotenine. Bufotenine binds to the 5-HT2A receptor with much higher affinity than 5-MeO-DMT itself.

5-MeO-DMT and compositions comprising 5-MeO-DMT besides other active components have hitherto only been used in the ritual or recreational context (erowid.org/chemicals/5meo_dmt/ 5meo_dmt_dose.shtml and erowid.org/chemicals/5meo_dmt/5meo_dmt_effects.shtml, accessed March 1, 2018). A recent internet survey which aimed to examine patterns of use, motivations for consumption, subjective effects, and potential benefits and consequences associated with the use of 5-MeO-DMT and compositions comprising 5-MeO-DMT besides other active ingredients, described that the majority of respondents with self-reported psychiatric conditions, including anxiety, depression, substance use problems, and post-traumatic stress disorder, perceived improvements in symptoms related to those disorders (Davis AK et al., J Psychopharmacol. 2018 Jul;32(7):779-792). However, as noted by the authors, "this study is cross-sectional, lacked a validated measure of psychiatric symptoms and assessment of prior psychiatric treatment, included many polysubstance users, which limits any causal inferences in the relation between the use of 5-MeO-DMT and an improvement in symptoms, and thus the associations of psychiatric benefits remain observational".

Application of 5-MeO-DMT together with a MAO inhibitor leads to an enhanced and prolonged drug effect but can also lead to more toxicity. Most commonly described routes of administration for 5-MeO-DMT in the ritual or recreational context are inhalation of smoke or vapor or intranasal insufflation, but other routes such as intravenous, rectal or oral application have also been described, with absorption via the latter route being limited by a substantial first-pass effect, probably through the rapid action of MAO enzymes in the gut and liver.

### Advantageous Effects of Invention

The advantageous effects of the invention, as compared to the current standard of care in the respective indication include, but are not limited to: a) a larger percentage of patients experiencing a clinical response, b) a larger average clinical response, c) an earlier onset of the clinical response, d) a more durable clinical response, e) a similar or better clinical response with fewer or different side effects and therefore improved compliance, and f) a similar or better clinical response with a more convenient therapeutic regimen with fewer drug administrations and therefore improved compliance. As compared to the previously studied psychedelics the invention can additionally provide, for example, for g) a similar or better clinical response with a shorter duration of the acute psychoactive effects after dosing and therefore improved convenience and compliance, h) an improved dosing regimen and administration route with higher propensity for and better reproducibility of achievement of peak psychedelic experiences, while avoiding unnecessary high doses and associated side effects, and with better tolerability, and therefore improved compliance. It is further noted that the advantageous effects are in particular achieved in treatment-resistant patients as defined herein.

### Definitions

As used in the context of the present invention, unless otherwise noted, the term "5-MeO-DMT" refers to the free base 5-MeO-DMT. It is contemplated that pharmaceutically acceptable salts of 5-MeO-DMT will typically be used. An example for such a salt is the hydrochloride. The appropriate weight amount of a salt to be administered can be calculated from the weight amount of the free base, assuming that equimolar amounts are used.

As used in the context of the present invention, a "patient" to be treated is a human subject who is diagnosed with major depressive disorder by a licensed professional in accordance with accepted medical practice. Diagnosis can, for instance, be in accordance with the Diagnostic and Statistical Manual of Mental Disorders - Fifth Edition (DSM-5) published by the American Psychiatric Association as suffering from a mental disorder, in particular major depressive disorder, persistent depressive disorder, anxiety disorder, posttraumatic stress disorder, body dysmorphic disorder, obsessive-compulsive disorder, eating disorder, or psychoactive substance abuse. The diagnosis will be by a physician or a psychologist. It is not sufficient that the human subject himself considers that he is suffering from one of the disorders.

As used the context of the present invention, "suicidal ideation" refers to thinking about, considering, or planning for suicide. The presence of suicidal ideation in a patient will be diagnosed by a physician or a psychologist, using established protocols and methods for diagnosing suicidality. It is generally not sufficient that the patient himself considers that he is suffering from suicidal ideation. In some situations, a patient experiencing suicidal ideation will be at imminent risk of committing suicide, or will be considered to have 'intent to act.'

As used in the context of the present invention, unless otherwise noted, the terms "treating" and "treatment" shall include the management and care of a patient for the purpose of combating a disease, condition, or disorder and includes the administration of compounds and methods according to present invention to alleviate the signs and/or symptoms or eliminate the disease, condition, or disorder.

As used in the context of the present invention, unless otherwise noted, the term "therapeutically effective amount" shall mean the amount of active compound or pharmaceutical ingredient that elicits the biological or clinical response in a human that is being sought by a researcher, medical doctor or other clinician, which includes alleviation of the signs and/or symptoms of the disease, condition or disorder being treated.

"Clinical response" includes, but is not limited to, improvements on rating scales such as the Clinical Global Impression - Severity scale (CGI-S), the Patient Global Impression - Severity scale (PGI-S), the Clinical Global Impression - Improvement scale (CGI-I) or the Patient Global Impression - Improvement scale (PGI-I) and further includes, but is not limited to, endpoints such as the Montgomery-Asberg Depression Rating Scale (MADRS) or the 17-item Hamilton Depression Rating Scale (HAM-D) for major depressive disorder and persistent depressive disorder, anxiety symptoms e.g. as measured by the Beck Anxiety Inventory (BAI), the Hamilton Anxiety Scale (HAM-A) or the State-Trait Anxiety Inventory (STAI) for anxiety disorder, the Clinician-Administered Posttraumatic Stress Disorder Scale (CAPS) for posttraumatic stress disorder, the Yale-Brown Obsessive Compulsive Scale Modified for Body Dysmorphic Disorder (BDD-YBOCS) for body dysmorphic disorder, the Yale-Brown Obsessive Compulsive Scale (Y-BOCS) for obsessive-compulsive disorder, weight gain for anorexia nervosa, frequency of binge-purge episodes for bulimia nervosa, frequency of binge episodes for binge eating disorder, duration of abstinence or reduced substance use in psychoactive substance abuse and suicidality rating scales such as the Columbia-Suicide Severity Rating Scale (C-SSRS) or the suicidal thoughts item of the MADRS for suicidal ideation or the Clinical Global Impression - Severity of Suicidality - Revised (CGI-SS-R) scale (the CGI-SS-R is derived from the CGI-S, and is scored 0 = Normal, Not At All Suicidal; 1 = Questionably Suicidal; 2= Mildly Suicidal; 3 = Moderately Suicidal; 4 = Markedly Suicidal; 5 = Severely Suicidal; 6 = Extremely Suicidal). When assessing a clinical response at an early timepoint after drug administration (e.g. at 2 hours) based on endpoints which have been developed for a longer recall period (e.g. normally 7 days for the MADRS), a rational modification of such endpoint (e.g. changing the MADRS recall period to 2 hours and carrying forward the sleep item recorded at baseline before drug administration) may be applied.

As used in the context of the present invention, unless otherwise noted, the term "administration" (or "application") shall mean the introduction of an amount, which may be a predetermined amount, of active compound or pharmaceutical ingredient into a patient via any route. The active compound is administered by intravenous administration, by intramuscular administration or by subcutaneous administration.

As used in the context of the present invention, unless otherwise noted, the terms "dose" and "dosage" and "dosage amount" shall mean the amount of active compound or pharmaceutical ingredient which is administered to a patient in an individual administration. The term "dosage regimen" (or "dosing regimen") shall mean a defined sequence of one or more individual administrations.

### Aspects of the invention

The present invention is directed to improved methods for the treatment of major depressive disorder, as defined in the claims. The present disclosure also relates to improved methods for the treatment of mental disorders, in particular major depressive disorder, persistent depressive disorder, anxiety disorder, posttraumatic stress disorder, body dysmorphic disorder, obsessive-compulsive disorder, eating disorder and psychoactive substance abuse comprising administering to a patient as defined herein a therapeutically effective amount of 5-MeO-DMT or a pharmaceutically acceptable salt thereof. Treatment of these disorders includes, but is not limited to, the use of 5-MeO-DMT or a pharmaceutically acceptable salt thereof in patients as defined herein with such a disorder and suicidal ideation. Treatment of these disorders specifically includes the use of 5-MeO-DMT or a pharmaceutically acceptable salt thereof in patients as defined herein with a treatment-resistant form of a mentioned disorder. As used in the context of the present invention, "treatment-resistant" is defined for major depressive disorder and persistent depressive disorder as no adequate improvement to at least two adequate courses of pharmacological therapy in the current episode of depression, for anxiety disorder, posttraumatic stress disorder, body dysmorphic disorder and obsessive-compulsive disorder as no adequate improvement to at least one adequate course of pharmacological therapy and at least one adequate course of psychotherapy, and for eating disorder and psychoactive substance abuse as no adequate improvement after at least one adequate course of psychotherapy with or without pharmacological therapy. Whether an improvement is adequate is assessed in terms of clinical response and whether a therapy course is adequate is assessed in terms of regimen, dose, duration, and compliance. Adequateness is assessed and documented by a physician or psychologist using a defined set of criteria and it is not sufficient that the patient himself considers that a prior therapy course was not adequate or that he not adequately responded. Adequateness can be assessed retrospectively and prospectively.

The treatment of patients with anxiety disorder includes, but is not limited to, patients with panic disorder, phobic anxiety disorders, social anxiety disorder and generalized anxiety disorder. The treatment of patients with eating disorder includes but is not limited to anorexia nervosa, bulimia nervosa and binge eating disorder. The treatment of patients with substance abuse includes, but is not limited to, patients with alcohol related disorders, opioid related disorders, sedative, hypnotic, or anxiolytic related disorders, cocaine related disorders, other stimulant related disorders and nicotine dependence.

Disorders preferably treated according to the disclosure are major depressive disorder, generalized anxiety disorder, obsessive compulsive-disorder and anorexia nervosa including their treatment-resistant forms. The disorder treated according to the invention is major depressive disorder including its treatment-resistant form, and including patients with major depressive disorder having suicidal ideation. In an embodiment the dosage amount of 5-MeO-DMT administered to a patient, as defined herein, with a mental disorder, in particular major depressive disorder, persistent depressive disorder, anxiety disorder, posttraumatic stress disorder, body dysmorphic disorder, obsessive-compulsive disorder, eating disorder and psychoactive substance abuse, including a treatment-resistant form of these disorders, and including these disorders associated with suicidal ideation, is in the range of about 1 mg to about 10 mg, or any amount of range therein. Useful specific amounts are e.g. about 1 mg, about 2 mg, about 3 mg, about 4 mg, about 5 mg, about 6 mg, about 7 mg, about 8 mg, about 9 mg and about 10 mg. It is understood that a pharmaceutically acceptable salt of 5-MeO-DMT is preferably used in this embodiment, and that the appropriate weight amounts of the salt to be administered can be calculated from the stated weight amounts of the free base, assuming that equimolar amounts are used.

Note that in this specification, when ranges are set forth, such as "about 1 mg to about 10 mg," the inventor contemplates all discrete values within that range, some of which are specifically mentioned, but all of which are not - simply for the purpose of brevity.

In preferred embodiments the improved methods for the treatment of a patient, as defined herein, with a mental disorder, in particular major depressive disorder, persistent depressive disorder, anxiety disorder, posttraumatic stress disorder, body dysmorphic disorder, obsessive-compulsive disorder, eating disorder and psychoactive substance abuse, including a treatment-resistant form of these disorders, and including these disorders associated with suicidal ideation, with a therapeutically effective amount of 5-MeO-DMT, comprise the occurrence of a clinical response not later than about 2 hours after administration of 5-MeO-DMT.

In preferred embodiments the improved methods for the treatment of a patient, as defined herein, with a mental disorder, in particular major depressive disorder, persistent depressive disorder, anxiety disorder, posttraumatic stress disorder, body dysmorphic disorder, obsessive-compulsive disorder, eating disorder and psychoactive substance abuse, including a treatment-resistant form of these disorders, and including these disorders associated with suicidal ideation, with a therapeutically effective amount of 5-MeO-DMT, comprise the persistence of a clinical response, including a clinical response which occurred not later than about 2 hours after administration of 5-MeO-DMT, until at least about 6 days after the last administration of 5-MeO-DMT, preferably until at least about 14 days after the last administration of 5-MeO-DMT, more preferably until at least about 28 days after the last administration of 5-MeO-DMT.

In preferred embodiments the improved methods for the treatment of a patient, as defined herein, with a mental disorder, in particular major depressive disorder, persistent depressive disorder, anxiety disorder, posttraumatic stress disorder, body dysmorphic disorder, obsessive-compulsive disorder, eating disorder and psychoactive substance abuse, including a treatment-resistant form of these disorders, and including these disorders associated with suicidal ideation, with a therapeutically effective amount of 5-MeO-DMT comprise the administration of more than a single dose of 5-MeO-DMT.

In a preferred embodiment this more than a single dose of 5-MeO-DMT is administered to a patient in one or more treatment blocks, each block consisting of 2 to 7 administrations, with not less than about 1 hour and not more than about 24 hours between each administration within each treatment block, and not less than about 6 days between the end of one treatment block and the start of the next treatment block.

In an even more preferred embodiment this more than a single dose of 5-MeO-DMT is administered to a patient in one or more treatment blocks, each block consisting of 1 to 3 administrations, with about 24 hours between each administration within each treatment block, and not less than about 6 days between the end of one treatment block and the start of the next treatment block.

In a most preferred embodiment this more than a single dose of 5-MeO-DMT is administered to a patient in one or more treatment blocks, each block consisting of 1 to 3 administrations, with about 2 to 4 hours between each administration within each treatment block, and not less than about 6 days between the end of one treatment block and the start of the next treatment block.

In an embodiment the dosage amount of the 5-MeO-DMT administered to an individual patient in each of the administrations and in each of the treatment blocks is constant for that individual patient and is selected from about 1 mg to about 10 mg.

In a preferred embodiment the dosage amount of the 5-MeO-DMT administered to an individual patient is selected from about 1 mg to about 2 mg for the first administration within each treatment block, and then increases with each subsequent administration within each treatment block until the earlier of 10 mg being reached or all administrations within that treatment block being administered.

In an even more preferred embodiment the dosage amount of the 5-MeO-DMT administered to an individual patient is selected from about 1 mg to about 2 mg for the first administration within each treatment block, and then increases with each subsequent administration within each treatment block until the earlier of 10 mg being reached or all administrations within that treatment block being administered or the patient having experienced a peak psychedelic experience or the supervising physician having decided that further dose increases are inappropriate based on observed side effects.

For embodiments where the dosage amount increases for subsequent administrations, the dosage amount for the next administration is determined by adding about 0.25 mg to about 3 mg, preferably about 0.5 mg to about 3 mg, to the dosage amount of the prior administration. For example, if the dosage amount of the first administration was 1 mg and the dosage amount increase is 3 mg, unless one of the previously mentioned stopping criteria has been reached, then the dosage amount of the second administration will be 4 mg. Preferably, the dosage amount for the third administration will be 7 mg.

In a preferred embodiment the dosage amount of the 5-MeO-DMT administered to an individual patient in each treatment block is selected from about 1 mg to about 3 mg for the first administration, and then increased, unless the patient has already experienced a peak psychedelic experience within that treatment block or the supervising physician has decided that further dose increases are inappropriate based on observed side effects, to a dosage selected from about 4 mg to about 6 mg for the second administration, and from about 7 mg to about 9 mg for the third administration. Useful specific amounts for the first, second and third administration are e.g. about 2 mg, about 5 mg, and about 8 mg.

In a further preferred embodiment the dosage amount of the 5-MeO-DMT administered to an individual patient is selected from about 1 mg to about 2 mg for the first administration of the first treatment block, and then increases with each subsequent administration within that first treatment block until the earlier of 10 mg being reached or all administrations within that treatment block being administered or the patient having experienced a peak psychedelic experience or the supervising physician having decided that further dose increases are inappropriate based on observed side effects, with that highest dosage in that first treatment block being used as the dosage for all subsequent treatment blocks and administrations within those subsequent treatment blocks. For example, if the highest dosage in the first treatment block was 8 mg because the patient experienced a peak psychedelic experience at that dose, then the dosage for all subsequent treatment blocks and administrations within those subsequent treatment blocks will be 8 mg.

In a most preferred embodiment the dosage amount of the 5-MeO-DMT administered to an individual patient is selected from about 1 mg to about 3 mg for the first administration of the first treatment block, and then increased, unless the patient has already experienced a peak psychedelic experience within that treatment block or the supervising physician has decided that further dose increases are inappropriate based on observed side effects, to a dosage selected from about 4 mg to about 6 mg for the second administration of the first treatment block, and from about 7 mg to about 9 mg for the third administration of the first treatment block, with the highest dosage in that first treatment block being used as the dosage for all subsequent treatment blocks and administrations within those subsequent treatment blocks. Useful specific amounts for the first, second and third administration in the first treatment block are e.g. about 2 mg, about 5 mg, and about 8 mg.

It is understood that a pharmaceutically acceptable salt of 5-MeO-DMT is preferably used in all of the above dosing regimen, and that the appropriate weight amounts of the salt to be administered can be calculated from the stated weight amounts of the free base, assuming that equimolar amounts are used.

According to the invention, 5-MeO-DMT is preferably not administered together with a MAO inhibitor.

The occurrence of a "peak psychedelic experience" in a patient can be identified through achievement of at least 60% of the maximum possible score in each of the four subscales (mystical, positive mood, transcendence of time and space, and ineffability) of the 30-item revised Mystical Experience Questionnaire (MEQ30) (as described in Barrett FS, J Psychopharmacol. 2015;29(11):1182-90; Score items shown in Example 4).

The occurrence of a "peak psychedelic experience" in a patient can also be identified through achievement of at least 60% of the maximum possible score of the Oceanic Boundlessness (OBN) dimension of the Altered States of Consciousness (ASC) questionnaire (as described in Roseman L et al., Front Pharmacol. 2018; 8:974).

The occurrence of a "peak psychedelic experience" in a patient can also be identified through achievement of a score of at least 75 in the Peak Psychedelic Experience Questionnaire (PPEQ) Total Score, which averages answers scored by the patient from 0 to 100 for the following three questions: 1. How intense was the experience; 2. To what extent did you lose control; 3. How profound (i.e. deep and significant) was the experience?

The therapeutically effective amount of 5-MeO-DMT is administered by intravenous administration, by intramuscular administration or by subcutaneous. Administration via these routes can assure a rapid onset of action. A most preferred route of administration is administration via the intramuscular route, i.e. by intramuscular injection.

5-MeO-DMT can preferably be employed as a pharmaceutically acceptable salt in the form of a formulation for administration via injection, examples of excipients and vehicles for such formulations being known in the art.

The patient may also receive psychotherapeutic interventions.

According to the invention, the disease to be treated is major depressive disorder. In this same specific embodiment the patient may suffer from a treatment-resistant form of major depressive disorder. In this same specific embodiment the patient may also suffer from suicidal ideation with intent to act, and he may be considered at imminent risk for suicide. In this same specific embodiment the dosage amount of 5-MeO-DMT administered to a patient, as defined herein, with a major depressive disorder, is in the range from about 1 mg to about 10 mg, and is administered via intramuscular administration in the form of a formulation for administration based on a pharmaceutically acceptable salt of 5-MeO-DMT, which weight amount can be calculated from the stated weight amounts of the 5-MeO-DMT free base, assuming that equimolar amounts are used. Useful specific amounts of 5-MeO-DMT in this specific embodiment are, e.g., about 1 mg, about 2 mg, about 3 mg, about 4 mg, about 5 mg, about 6 mg, about 7 mg, about 8 mg, about 9 mg, and about 10 mg.

In this same specific embodiment, a clinical response, as assessed by at least 50% improvement of the MADRS or HAM-D score, compared to the respective score prior to administration of 5-MeO-DMT, or a clinical response as assessed as at least a score of "much improved" in CGI-I or PGI-I scores, occurs not later than about 2 hours after administration of 5-MeO-DMT.

In this same specific embodiment, a remission of depressive symptoms as assessed by a MADRS score equal or less than 10, or a HAM-D score equal or less than 7, occurs not later than about 2 hours after administration of 5-MeO-DMT.

In this same specific embodiment, a clinical response as assessed by at least 50% improvement of the MADRS or HAM-D score compared to the respective score prior to administration of 5-MeO-DMT, or a clinical response as assessed as at least a score of "much improved" in CGI-I or PGI-I scores, including a clinical response which occurred not later than about 2 hours after administration of 5-MeO-DMT, persists until at least about 6 days after the last administration of 5-MeO-DMT, preferably until at least about 14 days after the last administration of 5-MeO-DMT, more preferably until at least about 28 days after the last administration of 5-MeO-DMT.

According to the invention, the disease to be treated is major depressive disorder. In this same specific embodiment, the dosage amount of the 5-MeO-DMT administered to an individual patient is selected from about 1 mg to about 3 mg for the first administration of the first treatment block, and then increased, unless the patient has already experienced a peak psychedelic experience within that treatment block or the supervising physician has decided that further dose increases are inappropriate based on observed side effects, to a dosage selected from about 4 mg to about 6 mg for the second administration of the first treatment block, and from about 7 mg to about 9 mg for the third administration of the first treatment block, with the highest dosage in that first treatment block being used as the dosage for all subsequent treatment blocks and administrations within those subsequent treatment blocks. Useful specific amounts for the first, second and third administration in the first treatment block are e.g. about 2 mg, about 5 mg, and about 8 mg. In this same specific embodiment, the interval between each administration within the first treatment block is about 3 hours. In this same specific embodiment, the 5-MeO-DMT is administered via intramuscular administration in the form of a formulation for administration based on a pharmaceutically acceptable salt of 5-MeO-DMT, which weight amount can be calculated from the stated weight amounts of the 5-MeO-DMT free base, assuming that equimolar amounts are used. In this same specific embodiment, the occurrence of a peak psychedelic experience is identified through achievement of at least 60% of the maximum possible score in each of the four subscales (mystical, positive mood, transcendence of time and space, and ineffability) of the 30-item revised Mystical Experience Questionnaire (MEQ30) (as described in Barrett FS, J Psychopharmacol. 2015;29(11):1182-90) or by achievement of a Peak Psychedelic Experience Questionnaire (PPEQ) Total Score of at least 75.

According to the invention, the disease to be treated is major depressive disorder. In this same specific embodiment, the dosage amount of the 5-MeO-DMT administered to an individual patient in each treatment block is selected from about 1 mg to about 3 mg for the first administration, and then increased, unless the patient has already experienced a peak psychedelic experience within that treatment block or the supervising physician has decided that further dose increases are inappropriate based on observed side effects, to a dosage selected from about 4 mg to about 6 mg for the second administration, and from about 7 mg to about 9 mg for the third administration. Useful specific amounts for the first, second and third administration in the first treatment block are e.g. about 2 mg, about 5 mg, and about 8 mg. In this same specific embodiment, the interval between each administration within each treatment block is about 3 hours and the interval between the end of one treatment block and the start of the next treatment block is about 6 days. In this same specific embodiment, the 5-MeO-DMT is administered via intramuscular administration in the form of a formulation for administration based on a pharmaceutically acceptable salt of 5-MeO-DMT, which weight amount can be calculated from the stated weight amounts of the 5-MeO-DMT free base, assuming that equimolar amounts are used. In this same specific embodiment, the occurrence of a peak psychedelic experience is identified through achievement of at least 60% of the maximum possible score in each of the four subscales (mystical, positive mood, transcendence of time and space, and ineffability) of the 30-item revised Mystical Experience Questionnaire (MEQ30) (as described in Barrett FS, J Psychopharmacol. 2015;29(11):1182-90) or by achievement of a Peak Psychedelic Experience Questionnaire (PPEQ) Total Score of at least 75.

In a specific embodiment the disease to be treated is treatment-resistant major depressive disorder. In this same specific embodiment the patient may suffer from suicidal ideation with intent to act, and he may be considered at imminent risk for suicide. In this same specific embodiment, the dosage amount of the 5-MeO-DMT administered to an individual patient is selected from about 1 mg to about 3 mg for the first administration of the first treatment block, and then increased, unless the patient has already experienced a peak psychedelic experience within that treatment block or the supervising physician has decided that further dose increases are inappropriate based on observed side effects, to a dosage selected from about 4 mg to about 6 mg for the second administration of the first treatment block, and from about 7 mg to about 9 mg for the third administration of the first treatment block, with the highest dosage in that first treatment block being used as the dosage for all subsequent treatment blocks and administrations within those subsequent treatment blocks. Useful specific amounts for the first, second and third administration in the first treatment block are e.g. about 2 mg, about 5 mg, and about 8 mg. In this same specific embodiment, the interval between each administration within the first treatment block is about 3 hours. In this same specific embodiment, the 5-MeO-DMT is administered via intramuscular administration in the form of a formulation for administration based on a pharmaceutically acceptable salt of 5-MeO-DMT, which weight amount can be calculated from the stated weight amounts of the 5-MeO-DMT free base, assuming that equimolar amounts are used. In this same specific embodiment, the occurrence of a peak psychedelic experience is identified through achievement of at least 60% of the maximum possible score in each of the four subscales (mystical, positive mood, transcendence of time and space, and ineffability) of the 30-item revised Mystical Experience Questionnaire (MEQ30) (as described in Barrett FS, J Psychopharmacol. 2015;29(11):1182-90) or by achievement of a Peak Psychedelic Experience Questionnaire (PPEQ) Total Score of at least 75.

In a further specific embodiment the disease to be treated is treatment-resistant major depressive disorder. In this same specific embodiment, the dosage amount of the 5-MeO-DMT administered to an individual patient in each treatment block is selected from about 1 mg to about 3 mg for the first administration, and then increased, unless the patient has already experienced a peak psychedelic experience within that treatment block or the supervising physician has decided that further dose increases are inappropriate based on observed side effects, to a dosage selected from about 4 mg to about 6 mg for the second administration, and from about 7 mg to about 9 mg for the third administration. Useful specific amounts for the first, second and third administration in the first treatment block are e.g. about 2 mg, about 5 mg, and about 8 mg. In this same specific embodiment, the interval between each administration within each treatment block is about 3 hours and the interval between the end of one treatment block and the start of the next treatment block is about 6 days. In this same specific embodiment, the 5-MeO-DMT is administered via intramuscular administration in the form of a formulation for administration based on a pharmaceutically acceptable salt of 5-MeO-DMT, which weight amount can be calculated from the stated weight amounts of the 5-MeO-DMT free base, assuming that equimolar amounts are used. In this same specific embodiment, the occurrence of a peak psychedelic experience is identified through achievement of at least 60% of the maximum possible score in each of the four subscales (mystical, positive mood, transcendence of time and space, and ineffability) of the 30-item revised Mystical Experience Questionnaire (MEQ30) (as described in Barrett FS, J Psychopharmacol. 2015;29(11):1182-90) or by achievement of a Peak Psychedelic Experience Questionnaire (PPEQ) Total Score of at least 75.

### Embodiments

Specific embodiments of the present invention are listed below.

It is emphasized that embodiments and claims which describe that a clinical response a) occurs not later than a specified timepoint, b) persists until at least a specified timepoint, or c) is present at a specified timepoint "after the last administration of 5-MeO-DMT or a pharmaceutically acceptable salt thereof", include, through dependencies from other embodiments and claims, such as embodiments and claims where a) the "last administration" is actually the first administration of 5-MeO-DMT or a pharmaceutically acceptable salt thereof, and b) the "last administration" is actually the administration of 5-MeO-DMT or a pharmaceutically acceptable salt thereof, where the patient experienced a peak psychedelic experience, which can be the first administration of 5-MeO-DMT or a pharmaceutically acceptable salt thereof, but can also be a later administration of 5-MeO-DMT or a pharmaceutically acceptable salt thereof at a higher dose than the first administration of 5-MeO-DMT or a pharmaceutically acceptable salt thereof.

### Embodiments relating to the treatment of major depressive disorder

1. 5-Methoxy-N,N-dimethyltryptamine (5-MeO-DMT) or a pharmaceutically acceptable salt thereof for use in treating major depressive disorder in a patient who is diagnosed with major depressive disorder by a licensec professional in accordance with accepted medical practice.
2. 5-MeO-DMT or a pharmaceutically acceptable salt thereof for use as in embodiment 1, wherein the disorder is diagnosed in accordance with the Diagnostic and Statistical Manual of Mental Disorders - Fifth Edition (DSM-5) published by the American Psychiatric Association.
3. 5-MeO-DMT or a pharmaceutically acceptable salt thereof for use as in embodiments 1 or 2, wherein the patient suffers from moderate or severe major depressive disorder as indicated by a Montgomery-Åsberg Depression Rating Scale (MADRS) score of 20 or more or by a 17-item Hamilton Depression Rating Scale (HAM-D) score of 17 or more.
4. 5-MeO-DMT or a pharmaceutically acceptable salt thereof for use as in embodiment 3, wherein the patient suffers from severe major depressive disorder as indicated by a MADRS score of 35 or more or by a HAM-D score of 25 or more.
5. 5-MeO-DMT or a pharmaceutically acceptable salt thereof for use as in embodiments 1 to 4, wherein the patient is diagnosed with a treatment-resistant form of major depressive disorder.
6. 5-MeO-DMT or a pharmaceutically acceptable salt thereof for use as in embodiments 1 to 5, wherein the patient suffers in addition from suicidal ideation.
7. 5-MeO-DMT or a pharmaceutically acceptable salt thereof for use as in embodiment 6, wherein the patient suffers from suicidal ideation with intent to act.
8. 5-MeO-DMT or a pharmaceutically acceptable salt thereof for use as in embodiments 1 to 7, wherein the patient is at imminent risk for suicide.
9. 5-MeO-DMT or a pharmaceutically acceptable salt thereof for use as in embodiments 1 to 8, wherein the 5-MeO-DMT or salt thereof is administered at a dose or in a dosage regimen that causes the patient to experience a peak psychedelic experience.
10. 5-MeO-DMT or a pharmaceutically acceptable salt thereof for use as in embodiments 1 to 9, wherein a dosage of about 1 mg to about 10 mg 5-MeO-DMT is administered, or wherein equimolar amounts of the pharmaceutically acceptable salt are administered instead of 5-MeO-DMT.
11. 5-MeO-DMT or a pharmaceutically acceptable salt thereof for use as in embodiments 1 to 9, wherein a dosage of about 2 mg; or of about 5 mg; or of about 8 mg is administered, or wherein equimolar amounts of the pharmaceutically acceptable salt are administered instead of 5-MeO-DMT.
12. 5-MeO-DMT or a pharmaceutically acceptable salt thereof for use as in embodiments 1 to 10, wherein the 5-MeO-DMT or salt thereof is administered in a first dosage amount for a first administration; and the 5-MeO-DMT or salt thereof is administered in zero to six subsequent administrations; wherein each subsequent administration uses a dosage amount higher than the previous administration unless the patient experiences a peak psychedelic experience.
13. 5-MeO-DMT or a pharmaceutically acceptable salt thereof for use as in embodiments 1 to 12, wherein the 5-MeO-DMT is administered in a dosage from about 1 mg to about 3 mg for a first administration, and then increased, unless the patient has already experienced a peak psychedelic experience, to a dosage from about 4 mg to about 6 mg for a second administration, and then increased, unless the patient has already experienced a peak psychedelic experience, to a dosage from about 7 mg to about 9 mg for a third administration, or wherein equimolar amounts of the pharmaceutically acceptable salt are administered instead of 5-MeO-DMT.
14. 5-MeO-DMT or a pharmaceutically acceptable salt thereof for use as in embodiment 13, wherein the first dosage of 5-MeO-DMT is about 2 mg, the second dosage of 5-MeO-DMT is about 5 mg, and the third dosage of 5-MeO-DMT is about 8 mg; or wherein equimolar amounts of the pharmaceutically acceptable salt are administered instead of 5-MeO-DMT.
15. 5-MeO-DMT or a pharmaceutically acceptable salt thereof for use as in embodiments 12 to 14, wherein the interval between two administrations is not less than 1 hour and not more than 24 hours, such as about 2 to 4 hours.
16. 5-MeO-DMT or a pharmaceutically acceptable salt thereof for use as in embodiments 9 to 15, wherein the occurrence of a peak psychedelic experience is identified through achievement of at least 60% of the maximum possible score in each of the four subscales (mystical, positive mood, transcendence of time and space, and ineffability) of the 30-item revised Mystical Experience Questionnaire (MEQ30) or is identified through achievement of at least 60% of the maximum possible score of the Oceanic Boundlessness (OBN) dimension of the Altered States of Consciousness (ASC) questionnaire or is identified through achievement of a Peak Psychedelic Experience Questionnaire (PPEQ) Total Score of at least 75.
17. 5-MeO-DMT or a pharmaceutically acceptable salt thereof for use as in embodiment 16, wherein the occurrence of a peak psychedelic experience is identified through achievement of a Peak Psychedelic Experience Questionnaire (PPEQ) Total Score of at least 75.
18. 5-MeO-DMT or a pharmaceutically acceptable salt thereof for use as in any of the prior embodiments, wherein the 5-MeO-DMT or a pharmaceutically acceptable salt thereof is administered via intramuscular injection.
19. 5-MeO-DMT or a pharmaceutically acceptable salt thereof for use as in embodiments 1 to 18, wherein a clinical response, as assessed by at least a score of "much improved" in the Clinical Global Impression - Improvement (CGI-I) score or the Patient Global Impression - Improvement (PGI-I) score, occurs not later than about 2 hours after the last administration of 5-MeO-DMT or a pharmaceutically acceptable salt thereof.
20. 5-MeO-DMT or a pharmaceutically acceptable salt thereof for use as in embodiments 1 to 19, wherein the clinical response, as assessed by at least a score of "much improved" in the CGI-I score or the PGI-I score, persists until at least 6 days after the last administration of 5-MeO-DMT or a pharmaceutically acceptable salt thereof.
21. 5-MeO-DMT or a pharmaceutically acceptable salt thereof for use as in embodiments 1 to 20, wherein the clinical response, as assessed by at least a score of "much improved" in the CGI-I score or the PGI-I score, persists until at least 14 days after the last administration of 5-MeO-DMT or a pharmaceutically acceptable salt thereof.
22. 5-MeO-DMT or a pharmaceutically acceptable salt thereof for use as in embodiments 1 to 21, wherein the clinical response, as assessed by at least a score of "much improved" in the CGI-I score or the PGI-I score, persists until at least 28 days after the last administration of 5-MeO-DMT or a pharmaceutically acceptable salt thereof.
23. 5-MeO-DMT or a pharmaceutically acceptable salt thereof for use as in embodiments 1 to 22, wherein a clinical response, as assessed by at least 50% improvement of the MADRS or HAM-D score, compared to the respective score prior to treatment, occurs not later than about 2 hours after the last administration of 5-MeO-DMT or a pharmaceutically acceptable salt thereof.
24. 5-MeO-DMT or a pharmaceutically acceptable salt thereof for use as in embodiments 1 to 23, wherein a remission of depressive symptoms, as assessed by a MADRS score equal to or less than 10, or a HAM-D score equal to or less than 7, occurs not later than about 2 hours after the last administration of 5-MeO-DMT or a pharmaceutically acceptable salt thereof.
25. 5-MeO-DMT or a pharmaceutically acceptable salt thereof for use as in embodiments 1 to 24, wherein the clinical response, as assessed by at least 50% improvement of the MADRS or HAM-D score, compared to the respective score prior to treatment, persists until at least 6 days after the last administration of 5-MeO-DMT or a pharmaceutically acceptable salt thereof.
26. 5-MeO-DMT or a pharmaceutically acceptable salt thereof for use as in embodiments 1 to 25, wherein there is a clinical response, as assessed by at least 75% improvement of the MADRS or HAM-D score, compared to the respective score prior to treatment, on day 7 after the last administration of 5-MeO-DMT or a pharmaceutically acceptable salt thereof.
27. 5-MeO-DMT or a pharmaceutically acceptable salt thereof for use as in embodiments 1 to 26, wherein the patient is in remission of depressive symptoms, as assessed by a MADRS score equal to or less than 10, or a HAM-D score equal to or less than 7, on day 7 after the last administration of 5-MeO-DMT or a pharmaceutically acceptable salt thereof.
28. 5-MeO-DMT or a pharmaceutically acceptable salt thereof for use as in embodiments 1 to 27, wherein the clinical response, as assessed by at least 50% improvement of the MADRS or HAM-D score, compared to the respective score prior to treatment, persists until at least 14 days after the last administration of 5-MeO-DMT or a pharmaceutically acceptable salt thereof.
29. 5-MeO-DMT or a pharmaceutically acceptable salt thereof for use as in embodiments 1 to 28, wherein there is a clinical response, as assessed by at least 75% improvement of the MADRS or HAM-D score, compared to the respective score prior to treatment, on day 14 after the last administration of 5-MeO-DMT or a pharmaceutically acceptable salt thereof.
30. 5-MeO-DMT or a pharmaceutically acceptable salt thereof for use as in embodiments 1 to 29, wherein the patient is in remission of depressive symptoms, as assessed by a MADRS score equal to or less than 10, or a HAM-D score equal to or less than 7, on day 14 after the last administration of 5-MeO-DMT or a pharmaceutically acceptable salt thereof.
31. 5-MeO-DMT or a pharmaceutically acceptable salt thereof for use as in embodiments 1 to 30, wherein the clinical response, as assessed by at least 50% improvement of the MADRS or HAM-D score, compared to the respective score prior to treatment, persists until at least 28 days after the last administration of 5-MeO-DMT or a pharmaceutically acceptable salt thereof.
32. 5-MeO-DMT or a pharmaceutically acceptable salt thereof for use as in embodiments 1 to 31, wherein there is a clinical response, as assessed by at least 75% improvement of the MADRS or HAM-D score, compared to the respective score prior to treatment, on day 28 after the last administration of 5-MeO-DMT or a pharmaceutically acceptable salt thereof.
33. 5-MeO-DMT or a pharmaceutically acceptable salt thereof for use as in embodiments 1 to 32, wherein the patient is in remission of depressive symptoms, as assessed by a MADRS score equal to or less than 10, or a HAM-D score equal to or less than 7, on day 28 after the last administration of 5-MeO-DMT or a pharmaceutically acceptable salt thereof.
34. 5-MeO-DMT or a pharmaceutically acceptable salt thereof for use as in embodiments 1 to 33, wherein the 5-MeO-DMT or the pharmaceutically acceptable salt thereof is administered in one or more treatment blocks.
35. 5-MeO-DMT or a pharmaceutically acceptable salt thereof for use as in embodiment 34, wherein a subsequent treatment block is administered not less than about 6 days after the preceding treatment block.
36. 5-MeO-DMT or a pharmaceutically acceptable salt thereof for use as in embodiments 34 to 35, wherein the first treatment block involves administration of the 5-MeO-DMT or salt thereof as defined in embodiments 9 to 18.
37. 5-MeO-DMT or a pharmaceutically acceptable salt thereof for use as in embodiment 34 to 36, wherein the first treatment block involves 1 to 7 administrations of the dosage at which the patient experienced a peak experience, with not less than about 1 hour and not more than about 24 hours between each administration.
38. 5-MeO-DMT or a pharmaceutically acceptable salt thereof for use as in embodiments 34 to 37, wherein each treatment block involves 1 to 7 administrations with not less than about 1 hour and not more than about 24 hours between each administration within each treatment block.
39. 5-MeO-DMT or a pharmaceutically acceptable salt thereof for use as in embodiments 34 or 38, wherein the second and any subsequent treatment block involves one or more administrations of the highest dosage of 5-MeO-DMT or the pharmaceutically acceptable salt thereof as administered during the first treatment block.
40. 5-MeO-DMT or a pharmaceutically acceptable salt thereof for use as in embodiments 1 to 33, wherein the 5-MeO-DMT or the pharmaceutically acceptable salt thereof is administered in two or more treatment blocks; wherein in the first treatment block the 5-MeO-DMT or salt thereof is administered in a first dosage amount for a first administration; and the 5-MeO-DMT or salt thereof is administered in the first treatment block in zero to six subsequent administrations; wherein each subsequent administration uses a dosage amount higher than the previous administration unless the patient experiences a peak psychedelic experience; wherein the occurrence of a peak psychedelic experience is identified through achievement of at least 60% of the maximum possible score in each of the four subscales (mystical, positive mood, transcendence of time and space, and ineffability) of the 30-item revised Mystical Experience Questionnaire (MEQ30) or is identified through achievement of at least 60% of the maximum possible score of the Oceanic Boundlessness (OBN) dimension of the Altered States of Consciousness (ASC) questionnaire; wherein the interval between two administrations in the first treatment block is not less than about 1 hour and not more than about 24 hours; wherein the second and any subsequent treatment block involves a single administration of the highest dosage of 5-MeO-DMT or the pharmaceutically acceptable salt thereof as administered during the first treatment block; wherein a subsequent treatment block is administered not less than about 6 days after the preceding treatment block.
41. 5-MeO-DMT or a pharmaceutically acceptable salt thereof for use as in embodiments 1 to 33, wherein the 5-MeO-DMT or the pharmaceutically acceptable salt thereof is administered in two or more treatment blocks; wherein in the first treatment block the 5-MeO-DMT or salt thereof is administered in a first dosage amount for a first administration; and the 5-MeO-DMT or salt thereof is administered in the first treatment block in zero to six subsequent administrations; wherein each subsequent administration uses a dosage amount higher than the previous administration unless the patient experiences a peak psychedelic experience; wherein the occurrence of a peak psychedelic experience is identified through achievement of a Peak Psychedelic Experience Questionnaire (PPEQ) Total Score of at least 75; wherein the interval between two administrations in the first treatment block is not less than about 1 hour and not more than about 24 hours; wherein the second and any subsequent treatment block involves a single administration of the highest dosage of 5-MeO-DMT or the pharmaceutically acceptable salt thereof as administered during the first treatment block; wherein a subsequent treatment block is administered not less than about 6 days after the preceding treatment block.

The invention is defined by the claims.

### Embodiments relating to the treatment of major depressive disorder with suicidal ideation with intent or with imminent risk for suicide.

1. 5-Methoxy-N,N-dimethyltryptamine (5-MeO-DMT) or a pharmaceutically acceptable salt thereof for use in treating major depressive disorder in a patient who is diagnosed with major depressive disorder by a licensec professional in accordance with accepted medical practice, and who also has suicidal ideation with intent or has imminent risk for suicide, as diagnosed by a licensed professional in accordance with accepted medical practice, wherein the 5-MeO-DMT is administered via the intravenous, intramuscular or subcutaneous route.
2. 5-MeO-DMT or a pharmaceutically acceptable salt thereof for use as in embodiment 1, wherein the disorder is diagnosed in accordance with the Diagnostic and Statistical Manual of Mental Disorders - Fifth Edition (DSM-5) published by the American Psychiatric Association.
3. 5-MeO-DMT or a pharmaceutically acceptable salt thereof for use as in embodiments 1 or 2, wherein the patient suffers from moderate or severe major depressive disorder as indicated by a Montgomery-Åsberg Depression Rating Scale (MADRS) score of 20 or more or by a 17-item Hamilton Depression Rating Scale (HAM-D) score of 17 or more and is considered Moderately Suicidal, Markedly Suicidal, Severely Suicidal or Extremely Suicidal based on the Clinical Global Impression - Severity of Suicidality - Revised (CGI-SS-R) scale.
4. 5-MeO-DMT or a pharmaceutically acceptable salt thereof for use as in embodiment 3, wherein the patient suffers from severe major depressive disorder as indicated by a MADRS score of 35 or more or by a HAM-D score of 25 or more and is considered Markedly Suicidal, Severely Suicidal or Extremely Suicidal based on the Clinical Global Impression - Severity of Suicidality - Revised (CGI-SS-R) scale.
5. 5-MeO-DMT or a pharmaceutically acceptable salt thereof for use as in embodiments 1 to 4, wherein the patient is diagnosed with a treatment-resistant form of major depressive disorder.
6. 5-MeO-DMT or a pharmaceutically acceptable salt thereof for use as in embodiments 1 to 5, wherein the 5-MeO-DMT or salt thereof is administered at a dose or in a dosage regimen that causes the patient to experience a peak psychedelic experience.
7. 5-MeO-DMT or a pharmaceutically acceptable salt thereof for use as in embodiments 1 to 6, wherein a dosage of about 1 mg to about 10 mg 5-MeO-DMT is administered, or wherein equimolar amounts of the pharmaceutically acceptable salt are administered instead of 5-MeO-DMT.
8. 5-MeO-DMT or a pharmaceutically acceptable salt thereof for use as in embodiments 1 to 6, wherein a dosage of about 2 mg; or of about 5 mg; or of about 8 mg is administered, or wherein equimolar amounts of the pharmaceutically acceptable salt are administered instead of 5-MeO-DMT.
9. 5-MeO-DMT or a pharmaceutically acceptable salt thereof for use as in embodiments 1 to 7, wherein the 5-MeO-DMT or salt thereof is administered in a first dosage amount for a first administration; and the 5-MeO-DMT or salt thereof is administered in zero to six subsequent administrations; wherein each subsequent administration uses a dosage amount higher than the previous administration unless the patient experiences a peak psychedelic experience.
10. 5-MeO-DMT or a pharmaceutically acceptable salt thereof for use as in embodiments 1 to 9, wherein the 5-MeO-DMT is administered in a dosage from about 1 mg to about 3 mg for a first administration, and then increased, unless the patient has already experienced a peak psychedelic experience, to a dosage from about 4 mg to about 6 mg for a second administration, and then increased, unless the patient has already experienced a peak psychedelic experience, to a dosage from about 17 mg to about 9 mg for a third administration, or wherein equimolar amounts of the pharmaceutically acceptable salt are administered instead of 5-MeO-DMT.
11. 5-MeO-DMT or a pharmaceutically acceptable salt thereof for use as in embodiment 10, wherein the first dosage of 5-MeO-DMT is about 2 mg, the second dosage of 5-MeO-DMT is about 5 mg, and the third dosage of 5-MeO-DMT is about 8 mg; or wherein equimolar amounts of the pharmaceutically acceptable salt are administered instead of 5-MeO-DMT.
12. 5-MeO-DMT or a pharmaceutically acceptable salt thereof for use as in embodiments 9 to 11, wherein the interval between two administrations is not less than 1 hour and not more than 24 hours, such as about 2 to 4 hours.
13. 5-MeO-DMT or a pharmaceutically acceptable salt thereof for use as in embodiments 6 to 12, wherein the occurrence of a peak psychedelic experience is identified through achievement of at least 60% of the maximum possible score in each of the four subscales (mystical, positive mood, transcendence of time and space, and ineffability) of the 30-item revised Mystical Experience Questionnaire (MEQ30) or is identified through achievement of at least 60% of the maximum possible score of the Oceanic Boundlessness (OBN) dimension of the Altered States of Consciousness (ASC) questionnaire or is identified through achievement of a Peak Psychedelic Experience Questionnaire (PPEQ) Total Score of at least 75.
14. 5-MeO-DMT or a pharmaceutically acceptable salt thereof for use as in embodiment 13, wherein the occurrence of a peak psychedelic experience is identified through achievement of a Peak Psychedelic Experience Questionnaire (PPEQ) Total Score of at least 75.
15. 5-MeO-DMT or a pharmaceutically acceptable salt thereof for use as in any of the prior embodiments, wherein the 5-MeO-DMT or a pharmaceutically acceptable salt thereof is administered via intramuscular injection.
16. 5-MeO-DMT or a pharmaceutically acceptable salt thereof for use as in embodiments 1 to 15, wherein a clinical response, as assessed by at least a score of "much improved" in the Clinical Global Impression - Improvement (CGI-I) score or the Patient Global Impression - Improvement (PGI-I) score, occurs not later than about 2 hours after the last administration of 5-MeO-DMT or a pharmaceutically acceptable salt thereof.
17. 5-MeO-DMT or a pharmaceutically acceptable salt thereof for use as in embodiments 1 to 16, wherein the clinical response, as assessed by at least a score of "much improved" in the CGI-I score or the PGI-I score, persists until at least 6 days after the last administration of 5-MeO-DMT or a pharmaceutically acceptable salt thereof.
18. 5-MeO-DMT or a pharmaceutically acceptable salt thereof for use as in embodiments 1 to 17, wherein the clinical response, as assessed by at least a score of "much improved" in the CGI-I score or the PGI-I score, persists until at least 14 days after the last administration of 5-MeO-DMT or a pharmaceutically acceptable salt thereof.
19. 5-MeO-DMT or a pharmaceutically acceptable salt thereof for use as in embodiments 1 to 18, wherein the clinical response, as assessed by at least a score of "much improved" in the CGI-I score or the PGI-I score, persists until at least 28 days after the last administration of 5-MeO-DMT or a pharmaceutically acceptable salt thereof.
20. 5-MeO-DMT or a pharmaceutically acceptable salt thereof for use as in embodiments 1 to 19, wherein a clinical response, as assessed by at least 50% improvement of the MADRS or HAM-D score, and a reduction of at least one level on the CGI-SS-R scale, compared to the respective score prior to treatment, occurs not later than about 2 hours after the last administration of 5-MeO-DMT or a pharmaceutically acceptable salt thereof.
21. 5-MeO-DMT or a pharmaceutically acceptable salt thereof for use as in embodiments 1 to 20, wherein a remission of depressive symptoms and suicidal ideation, as assessed by a MADRS score equal to or less than 10, or a HAM-D score equal to or less than 7, and a score of Not At All Suicidal or Questionably Suicidal on the CGI-SS-R scale, occurs not later than about 2 hours after the last administration of 5-MeO-DMT or a pharmaceutically acceptable salt thereof.
22. 5-MeO-DMT or a pharmaceutically acceptable salt thereof for use as in embodiments 1 to 21, wherein the clinical response, as assessed by at least 50% improvement of the MADRS or HAM-D score, and a reduction of at least one level on the CGI-SS-R scale, compared to the respective score prior to treatment, persists until at least 6 days after the last administration of 5-MeO-DMT or a pharmaceutically acceptable salt thereof.
23. 5-MeO-DMT or a pharmaceutically acceptable salt thereof for use as in embodiments 1 to 22, wherein there is a clinical response, as assessed by at least 75% improvement of the MADRS or HAM-D score, and a reduction of at least two levels on the CGI-SS-R scale, compared to the respective score prior to treatment, on day 7 after the last administration of 5-MeO-DMT or a pharmaceutically acceptable salt thereof.
24. 5-MeO-DMT or a pharmaceutically acceptable salt thereof for use as in embodiments 1 to 23, wherein the patient is in remission of depressive symptoms and suicidal ideation, as assessed by a MADRS score equal to or less than 10, or a HAM-D score equal to or less than 7, and a score of Not At All Suicidal or Questionably Suicidal on the CGI-SS-R scale, on day 7 after the last administration of 5-MeO-DMT or a pharmaceutically acceptable salt thereof.
25. 5-MeO-DMT or a pharmaceutically acceptable salt thereof for use as in embodiments 1 to 24, wherein the clinical response, as assessed by at least 50% improvement of the MADRS or HAM-D score, and a reduction of at least one level on the CGI-SS-R scale, compared to the respective score prior to treatment, persists until at least 14 days after the last administration of 5-MeO-DMT or a pharmaceutically acceptable salt thereof.
26. 5-MeO-DMT or a pharmaceutically acceptable salt thereof for use as in embodiments 1 to 25, wherein there is a clinical response, as assessed by at least 75% improvement of the MADRS or HAM-D score, and a reduction of at least two levels on the CGI-SS-R scale, compared to the respective score prior to treatment, on day 14 after the last administration of 5-MeO-DMT or a pharmaceutically acceptable salt thereof.
27. 5-MeO-DMT or a pharmaceutically acceptable salt thereof for use as in embodiments 1 to 26, wherein the patient is in remission of depressive symptoms and suicidal ideation, as assessed by a MADRS score equal to or less than 10, or a HAM-D score equal to or less than 7, and a score of Not At All Suicidal or Questionably Suicidal on the CGI-SS-R scale, on day 14 after the last administration of 5-MeO-DMT or a pharmaceutically acceptable salt thereof.
28. 5-MeO-DMT or a pharmaceutically acceptable salt thereof for use as in embodiments 1 to 27, wherein the clinical response, as assessed by at least 50% improvement of the MADRS or HAM-D score, and a reduction of at least one level on the CGI-SS-R scale, compared to the respective score prior to treatment, persists until at least 28 days after the last administration of 5-MeO-DMT or a pharmaceutically acceptable salt thereof.
29. 5-MeO-DMT or a pharmaceutically acceptable salt thereof for use as in embodiments 1 to 28, wherein there is a clinical response, as assessed by at least 75% improvement of the MADRS or HAM-D score, and a reduction of at least two levels on the CGI-SS-R scale, compared to the respective score prior to treatment, on day 28 after the last administration of 5-MeO-DMT or a pharmaceutically acceptable salt thereof.
30. 5-MeO-DMT or a pharmaceutically acceptable salt thereof for use as in embodiments 1 to 29, wherein the patient is in remission of depressive symptoms and suicidal ideation, as assessed by a MADRS score equal to or less than 10, or a HAM-D score equal to or less than 7, and a score of Not At All Suicidal or Questionably Suicidal on the CGI-SS-R scale, on day 28 after the last administration of 5-MeO-DMT or a pharmaceutically acceptable salt thereof.
31. 5-MeO-DMT or a pharmaceutically acceptable salt thereof for use as in embodiments 1 to 30, wherein the 5-MeO-DMT or the pharmaceutically acceptable salt thereof is administered in one or more treatment blocks.
32. 5-MeO-DMT or a pharmaceutically acceptable salt thereof for use as in embodiment 31, wherein a subsequent treatment block is administered not less than about 6 days after the preceding treatment block.
33. 5-MeO-DMT or a pharmaceutically acceptable salt thereof for use as in embodiments 31 to 32, wherein the first treatment block involves administration of the 5-MeO-DMT or salt thereof as defined in embodiments 9 to 18.
34. 5-MeO-DMT or a pharmaceutically acceptable salt thereof for use as in embodiment 31 to 33, wherein the first treatment block involves 1 to 7 administrations of the dosage at which the patient experienced a peak experience, with not less than about 1 hour and not more than about 24 hours between each administration.
35. 5-MeO-DMT or a pharmaceutically acceptable salt thereof for use as in embodiments 31 to 34, wherein each treatment block involves 1 to 7 administrations with not less than about 1 hour and not more than about 24 hours between each administration within each treatment block.
36. 5-MeO-DMT or a pharmaceutically acceptable salt thereof for use as in embodiments 31 or 35, wherein the second and any subsequent treatment block involves one or more administrations of the highest dosage of 5-MeO-DMT or the pharmaceutically acceptable salt thereof as administered during the first treatment block.
37. 5-MeO-DMT or a pharmaceutically acceptable salt thereof for use as in embodiments 1 to 30, wherein the 5-MeO-DMT or the pharmaceutically acceptable salt thereof is administered in two or more treatment blocks; wherein in the first treatment block the 5-MeO-DMT or salt thereof is administered in a first dosage amount for a first administration; and the 5-MeO-DMT or salt thereof is administered in the first treatment block in zero to six subsequent administrations; wherein each subsequent administration uses a dosage amount higher than the previous administration unless the patient experiences a peak psychedelic experience; wherein the occurrence of a peak psychedelic experience is identified through achievement of at least 60% of the maximum possible score in each of the four subscales (mystical, positive mood, transcendence of time and space, and ineffability) of the 30-item revised Mystical Experience Questionnaire (MEQ30) or is identified through achievement of at least 60% of the maximum possible score of the Oceanic Boundlessness (OBN) dimension of the Altered States of Consciousness (ASC) questionnaire; wherein the interval between two administrations in the first treatment block is not less than about 1 hour and not more than about 24 hours; wherein the second and any subsequent treatment block involves a single administration of the highest dosage of 5-MeO-DMT or the pharmaceutically acceptable salt thereof as administered during the first treatment block; wherein a subsequent treatment block is administered not less than about 6 days after the preceding treatment block.
38. 5-MeO-DMT or a pharmaceutically acceptable salt thereof for use as in embodiments 1 to 30, wherein the 5-MeO-DMT or the pharmaceutically acceptable salt thereof is administered in two or more treatment blocks; wherein in the first treatment block the 5-MeO-DMT or salt thereof is administered in a first dosage amount for a first administration; and the 5-MeO-DMT or salt thereof is administered in the first treatment block in zero to six subsequent administrations; wherein each subsequent administration uses a dosage amount higher than the previous administration unless the patient experiences a peak psychedelic experience; wherein the occurrence of a peak psychedelic experience is identified through achievement of a Peak Psychedelic Experience Questionnaire (PPEQ) Total Score of at least 75; wherein the interval between two administrations in the first treatment block is not less than about 1 hour and not more than about 24 hours; wherein the second and any subsequent treatment block involves a single administration of the highest dosage of 5-MeO-DMT or the pharmaceutically acceptable salt thereof as administered during the first treatment block; wherein a subsequent treatment block is administered not less than about 6 days after the preceding treatment block.

### Examples

The following Examples are listed to aid understanding of the invention.

### Example 1 (Reference Example)

### Example 1A - Administration of 5-MeO-DMT via inhalation

### Volcano Medic Vaporization System

A 5-MeO-DMT aerosol was generated by volatilization of the drug by way of the Volcano Medic Vaporization System (Storz & Bickel, Germany). The device consists of a hot air generator and a detachable valve balloon from which the aerosol is inhaled by the patient. The hot air generator can generate temperatures adjustable between about 40°C to about 210°C, with an airflow rate of about 12 liters per minute. The central part of the device is the dosing capsule to which relevant doses of 5-MeO-DMT in an ethanol solution are applied and which is then applied into the filling chamber of the device, where it is heated via the hot air. The dosing capsules contain a small disc made of tightly packed stainless-steel wire mesh (called the drip pad or liquid pad). The bottom and the lid of the dosing capsules have holes, allowing airflow through the dosing capsules. The dosing capsules and drip pad have the following characteristics, based on measurements of 10 sample capsules:

### Example 1A, Table 1. Characteristics of dosing capsules and drip pads.

| **Item** | **Mean (standard deviation)¹** |
|---|---|
| Dosing capsule without lid (outer diameter) | 14.3 mm (0.03 mm) |
| Dosing capsule without lid (height) | 8.0 mm (0.03 mm) |
| Dosing capsule without lid (weight) | 236.3 mg (2.6 mg) |
| Dosing capsule with lid (weight) | 361.9 mg (2.6 mg) |
| Dosing capsule with lid and with drip pad (weight) | 1323.4 mg (52.5 mg) |
| Lid (outer diameter) | 14.4 mm (0.06 mm) |
| Lid (height) | 3.2 mm (0.03 mm) |
| Lid (weight) | 125.6 mg (0.8 mg) |
| Number of holes (lid) | 33 (0) |
| Number of holes (dosing capsule base) | 33 (0) |
| Diameter of holes in lid and base | 1138 µm (57 µm) |
| Drip pad (weight) | 961.9 mg (52.2 mg) |
| Stainless steel wire in drip pad (diameter) | 113 µm (12 µm) |
| Stainless steel wire in drip pad (length) | 1062.0 cm (55.8 cm) |
| Stainless steel wire in drip pad (calculated surface area) | 37.78 cm² (1.99 cm²) |
| Drip pad weight / length index (mg/cm) | 0.906 (0.013) |

All measurements show the mean and standard deviation for measurements of 10 capsules, except for the diameter of holes in lid and base, which is based on 40 measurements across 2 capsules and for diameter of the stainless steel wire in drip pad, which is based on 40 measurements in different locations.

### 5-MeO-DMT aerosol generation and administration

Step 1: A stock solution of 5-MeO-DMT free base in 100% ethanol is prepared in a volumetric flask, so that the target dosage of 5-MeO-DMT free base to be administered via inhalation to the volunteer or patient is contained in a solution volume of 200 µl. Typical target dosages are from 1 mg to 25 mg 5-MeO-DMT. E.g. for a target dosage of 18 mg 5-MeO-DMT, 90 mg of 5-MeO-DMT will be dissolved in 100% ethanol for a final solution volume of 1 ml. Aliquots of the stock solution can then be stored in vials until further use.

Step 2: 200 µl of the solution is transferred to a dosing capsule containing the drip pad (Storz & Bickel, Germany), and then the dosing capsule is closed with its lid.

Step 3: The dosing capsule filled with the 5-MeO-DMT ethanol solution is transferred to the filling chamber of a first Volcano Medic Vaporizer, which has been pre-heated with the temperature set at 55°C. Then the airflow of the vaporizer is switched on for 60 seconds at the pre-set rate of about 12 l/min. The heated air will flow through the dosing capsule, allowing the ethanol to evaporate, with the target dosage of 5-MeO DMT being left in the capsule, as a thin layer covering the stainless-steel wire mesh. Accurate preparation of the dosing capsule can be confirmed by demonstrating that the final weight increase of the capsule compare to the weight of the empty capsule is about equal to the target dosage of 5-MeO-DMT.

Step 4: The prepared dosing capsule is removed from the filling chamber. It is then transferred to the filling chamber of a second Volcano Medic Vaporizer, which has been pre-heated with the temperature set at 210°C and the airflow on for at least 5 minutes and then turned off immediately prior to transfer. An inhalation balloon with a valve (Storz & Bickel, Germany) is mounted on the socket of the filling chamber, the filling chamber is closed tightly and immediately afterwards the airflow is switched on for exactly 15 seconds at the pre-set flow rate of about 12 l/min, and then turned off. This will allow the full dose of 5-MeO-DMT to aerosolize and be distributed in approximately 3 liters of air in the inhalation balloon. Accurate aerosolization of the 5-MeO-DMT can be confirmed by demonstrating that the capsule weight has returned to about its initial weight.

Step 5: The balloon is then disconnected from the filling chamber, which automatically closes the valve. After attachment of the mouthpiece to the balloon, the aerosol is ready for immediate administration to the volunteer or patient.

Step 6: To prepare for the administration, the patient is asked to initially perform 1-2 deep inhalations with full exhalations, ending this sequence with a deep exhalation. Then, with the mouthpiece firmly held against the lips, the full and complete volume of the inhalation balloon is inhaled in one inhalation, holding the breath for 10 (±2.5) seconds, followed by a normal exhalation. After completing the inhalation procedure, the patient will be instructed to lie down.

### Example 1B. Preparation of starting material

5-MeO-DMT (2.0 g) was dissolved in MTBE (4 mL, 2.0 volumes) at 35 to 40°C before being cooled to room temperature over 30 minutes. After stirring at room temperature for 50 minutes no crystallisation was observed, therefore, the batch temperature was decreased to 7 to 12°C over 30 minutes. After stirring at 7 to 12°C for 10 minutes crystallisation occurred. The batch was subsequently filtered following a 1 hour stir out at 7 to 12°C. After washing with MTBE (1 mL, 0.5 volumes), at 7 to 12°C, the batch was pulled dry under vacuum for 3.5 hours to yield a pale orange solid in 1.02 g (50% recovery). The isolated solid was analysed for purity by HPLC. The purity was found to be 99.74 %area.

The table below displays the impurity profile of isolated material.

### Example 1B, Table 1. Impurity profile of isolated material.

| **Impurity Profile** | | **HPLC Purity (area%)** | |
|---|---|---|---|
| | **RRT** | **Raw Material** | **Isolated Material** |
| | 0.87 | 0.07 | 0.06 |
| | 0.90 | 0.04 | 0.02 |
| | 0.92 | 0.03 | - |
| 5-MeO-DMT | 1.00 | **99.21** | **99.74** |
| | 1.18 | 0.13 | 0.04 |
| | 1.24 | 0.15 | 0.02 |
| | 1.28 | 0.02 | <0.01 |
| | 1.64 | - | 0.02 |
| | 1.67 | - | <0.01 |
| | 1.72 | - | - |
| | 1.96 | 0.02 | - |
| | 2.08 | - | - |
| | 2.11 | - | - |
| | 2.34 | 0.03 | - |
| | 2.38 | 0.29 | 0.08 |
| | 2.42 | - | - |
| | 2.61 | - | - |
| | 2.76 | 0.01 | - |
| | 2.82 | - | - |
| | 2.90 | - | - |

The results from the analysis indicated that the overall purity of the material was increased and the impurities at RRT 1.18 and at RRT 1.24 were purged to below 0.10%. The impurity at RRT 2.38 was also reduced to below the target of NMT 0.10%.

Solvent analysis of sample indicated an MTBE level of 17 ppm against an expected limit of NMT 5000 ppm.

### Example 2 (Reference Example)

### Clinical trial of 5-MeO-DMT administered via inhalation to patients with treatment-resistant major depressive disorder

### Study design:

A clinical trial was performed in which 5-MeO-DMT free base (purity not less than 99%) was administered to patients with treatment-resistant major depressive disorder (TRD). The objectives of the study were to assess the safety and tolerability, the dose-related psychedelic effects and the effects on various measures of depression of single-day dosing of 5-MeO-DMT. The trial was reviewed and approved by the relevant national competent authority and local medical ethics committee. Patients recruited into the trial had to meet the Diagnostic and Statistical Manual of Mental Disorders - Fifth Edition (DSM-5) diagnostic criteria for single-episode or recurrent major depressive disorder and had to be treatment-resistant, both aspects as evaluated by a psychiatrist or registered psychologist. Patients who were using any psychoactive medication or substance (such as anti-depressive medication) had to discontinue such medication or substance and had to observe a wash-out period before the 5-MeO-DMT administration. On the administration day, a single dose of 12 mg 5-MeO-DMT was administered to the patients via a single inhalation as described in Example 1A. Patients were closely monitored for 3.5 hours after administration, with additional follow-up visits 1 day and 7 days after dosing. Various safety related measures (e.g., adverse event reporting, safety laboratory analyses, vital signs, electrocardiogram (ECG), Clinician Administered Dissociative States Scale (CADSS), Psychomotor Vigilance Test (PVT), Digit Symbol Substitution Test (DSST)), measures of the intensity of the psychedelic effects (e.g., Peak Pyschedelic Experience Questionnaire (PPEQ), Altered States of Consciousness (ASC) Questionnaire, Mystical Experience Questionnaire (MEQ30), subjective description of the psychedelic experience), measures of depression severity (Montgomery-Åsberg Depression Rating Scale (MADRS), Patient's Global Impression of Severity scale (PGI-S), Patient's Global Impression of Improvement scale (PGI-I)), and additional psychological measures (e.g., Columbia-Suicide Severity Rating Scale (C-SSRS), Brief Psychiatric Rating Scale (BPRS)) were recorded at different time points. Psychological support via a psychologist and/or psychiatrist was available to the patients throughout the study, but no specific psychotherapy was applied, and no additional psychoactive medication was provided.

### Patient characteristics:

Two patients with major depressive disorder diagnosed by a psychiatrist according to DSM-5 diagnostic criteria were recruited into the study (Example 2, Table 1). The patients were considered treatment-resistant because they had not shown adequate improvement to at least two adequate courses of pharmacological therapy in the current episode of depression.

### Example 2, Table 1. Patient Characteristics

| | **Patient 1** | **Patient 2** |
|---|---|---|
| **Sex** | Female | Male |
| **Age** | 51 years | 25 years |
| **Inadequate response to adequate course of therapy (n)*** | 2 | 2 |

| | | |
|---|---|---|
| *Number of trials with an inadequate response (minimally improved; no change; minimally worse; much worse; very much worse; much or very much improved but relapsed on same regimen) to an adequate course of pharmacological therapy in the current episode of depression as assessed using the Antidepressant Treatment History Form - Short Form (ATHF-SF). | | |

### Results:

The patients completed all planned visit days. The inhalation procedure was adequately performed by the patients and was well tolerated with no inhalation-related adverse events. The first psychedelic symptoms occurred within a few seconds after the inhalation. The duration of the psychedelic experience as judged by an external observer was 16 min for patient 1 and 40 minutes for patient 2.

Except for a temporary, not clinically relevant increase in heart rate and blood pressure shortly after administration of 5-MeO-DMT, no other noteworthy changes in vital parameters occurred. Assessments of ECG (at 3 hours after administration), safety laboratory analyses (at 3 hours and 7 days), CADSS (at 3 hours, 1 day and 7 days) and cognitive tests (PVT, DSST; at 1 day and 7 days) were unremarkable. The few reported adverse events were mild, short-lasting and resolved spontaneously by the end of the study (Example 2, Table 2).

### Example 2, Table 2. Reported adverse events.

| | **Patient 1** | **Patient 2** |
|---|---|---|
| **Administration day** | Dizziness | *None reported* |
| **Day 1** | Headache, Flashback | Headache |
| **Day 7** | Headache | *None reported* |

All reported adverse events were mild and resolved spontaneously by the end of the study.

With regard to the intensity of the psychedelic experience, patient 1 fulfilled the criteria for a peak psychedelic experience based on all three assessed scores, while patient 2 fulfilled the criteria for the MEQ30 and PPEQ, but not for the ASC (Example 2, Table 3).

### Example 2, Table 3. Measures of the intensity of the psychedelic effects

| | | **Patient 1** | **Patient 2** |
|---|---|---|---|
| **MEQ30** | | | |
| | **Mystical (0 to 5)** | 3.5 | 3.1 |
| | **Positive Mood (0 to 5)** | 4.5 | 4.0 |
| | **Transcendence Time/Space (0 to 5)** | 3.7 | 3.5 |
| | **Ineffability (0 to 5)** | 4.7 | 3.3 |
| | **Total Score Average (0 to 5)** | 3.8 | 3.4 |
| | **PPE achieved¹** | Yes | Yes |

| **ASC** | | | |
|---|---|---|---|
| | **Oceanic Boundlessness (0-2700)** | 1696 | 1297 |
| | **PPE achieved²** | Yes | No |

| **PPEQ** | | | |
|---|---|---|---|
| | **Intensity (0-100)** | 94 | 100 |
| | **Loss of Control (0-100)** | 45 | 58 |
| | **Profoundness (0-100)** | 87 | 80 |
| | **PPEQ Total Score (0-100)** | 75.3 | 79.3 |
| | **PPE achieved³** | Yes | Yes |

| | | | |
|---|---|---|---|
| MEQ30, 30-item revised Mystical Experience Questionnaire; ASC, Altered States of Consciousness Questionnaire; PPEQ, Peak Psychedelic Experience Questionnaire; PPE, Peak Psychedelic experience. ¹Achievement of at least 60% of the maximum possible score in each of the four subscales (mystical, positive mood, transcendence of time and space and ineffability) of the MEQ30; ²Achievement of at least 60% of the maximum possible score of the Oceanic Boundlessness dimension of the ASC; ³Achievement of a PPEQ Total Score of at least 75. | | | |

Astonishingly, the patients reported a major improvement of their depressive symptoms as assessed by the MADRS, PGI-S and PGI-I already at the first assessment time point at 2 hours after drug administration, with the effect further deepening over time (Example 2, Table 4). The patients also fulfilled standard criteria for MADRS response (at least 50% improvement from baseline) and MADRS remission (MADRS total score equal or less than 10), rated "Normal, not at all ill" on the PGI-S and reported that their depressive symptoms had "very much improved" or "much improved" on the PGI-I at all assessment time points after drug administration, which is a highly surprising result.

The patients also improved on their ratings for suicidality after drug administration, as assessed using the suicidal thoughts item of the MADRS and the C-SSRS Suicidal ideation items. Other general psychiatric symptoms as assessed by the BPRS, e.g., somatic concern, anxiety, guilt and tension, also improved after the drug administration.

Importantly, patient 1 and patient 2 had a very similar intensity of psychedelic effects, which correlated with very similar anti-depressive clinical response. This observation supports that the occurrence of a peak experience is an important mechanism or at the least a surrogate behavioral marker for the underlying mechanism for the therapeutic efficacy of 5-MEO-DMT.

### Example 2, Table 4. Symptom scales at baseline, 2 hours, 1 day and 7 days after 5-MeO-DMT administration.

| | **Patient 1** | | | | **Patient 2** | | | | |
|---|---|---|---|---|---|---|---|---|---|
| | **B** | **2h** | **1d** | **7d** | **B** | **2h** | **1d** | **7d** | |
| **MADRS¹** | 31 | 10 | 8 | 3 | 33 | 9 | 5 | 5 | |
| **%MADRS change vs. baseline** | - | -68 | -74 | -90 | - | -73 | -85 | -85 | |
| **PGI-S²** | 5 | 1 | 1 | 1 | 5 | 1 | 1 | 1 | |
| **PGI-I³** | - | 1 | 1 | 1 | - | 2 | 2 | 2 | |
| **MADRS suicidal thoughts item⁴** | 1 | 0 | 0 | 0 | 1 | 0 | 1 | 0 | |
| **C-SSRS Suicidal Ideation⁵** | | Yes | No | No | No | No | No | No | No |
| **BPRS⁶** | | 42 | 27 | 18 | 18 | 45 | 26 | 19 | 18 |
| | **Somatic concern** | 3 | 2 | 1 | 1 | 4 | 1 | 1 | 1 |
| | **Anxiety** | 7 | 2 | 1 | 1 | 5 | 2 | 1 | 1 |
| | **Guilt feelings** | 5 | 1 | 1 | 1 | 5 | 1 | 1 | 1 |
| | **Tension** | 4 | 3 | 1 | 1 | 3 | 1 | 1 | 1 |
| | **Depressive Mood** | 6 | 2 | 1 | 1 | 5 | 3 | 1 | 1 |
| | **Unusual thought content** | 1 | 1 | 1 | 1 | 6 | 1 | 1 | 1 |

| | | | | | | | | | |
|---|---|---|---|---|---|---|---|---|---|
| B, Baseline; 2h, 2 hours; 1d, 1 day; 7d, 7 days; ¹The MADRS is a ten-item diagnostic questionnaire used to measure the severity of depressive episodes in patients with mood disorders, where each item yields a score of 0 to 6 and the overall score ranges from 0 to 60. Usual cut-off points are: 0 to 6 - normal/symptom absent, 7 to 19 - mild depression, 20 to 34 - moderate depression, >34 - severe depression; the recall period for MADRS at baseline was the last week, while the recall period at 2 hours, 1 day and 7 days after dosing spanned from the time point when the acute psychedelic effects after dosing have subsided to the assessment time point. At the 2-hour time point, the sleep item was not evaluated. Instead, the pre-dose MADRS score for the sleep item recorded at baseline before dosing was carried forward; ²The PGI-S for Depression is a 7-point scale that requires the patient to rate the severity of his/her depressive symptoms at the time of assessment. Possible ratings are: 1, Normal, not at all ill; 2, Borderline ill; 3, Mildly ill; 4, Moderately ill; 5, Markedly ill; 6, Severely ill; 7, Extremely ill; ³The PGI-I for Depression is a 7-point scale that requires the patient to assess how much his/her depressive symptoms have improved or worsened relative to a baseline state at the beginning of the intervention. The PGI-I scale is rated as comparison to baseline: 1, Very much improved; 2, Much improved; 3, Minimally improved; 4, No change; 5, Minimally worse; 6, Much worse; 7, Very much worse; ⁴The MADRS suicidal thoughts item scores suicidality of the patient and ranges from 0, Enjoys life and takes it as it comes to 6, Explicit plans for suicide when there is an opportunity. Active preparations for suicide; ⁵The C-SSRS is a detailed questionnaire assessing both suicidal behavior and suicidal ideation, the table shows whether any of the suicidal ideation items were present; ⁶The BPRS is intended to screen for the presence of various psychiatric symptoms in a structured fashion. 18 symptoms (somatic concern, anxiety, emotional withdrawal, conceptual disorganization, guilt feelings, tension, mannerisms and posturing, grandiosity, depressive mood, hostility, suspiciousness, hallucinatory behavior, motor retardation, uncooperativeness, unusual thought content, blunted affect, excitement and disorientation) are scored and each symptom is rated on a scale of 1-7. In addition to the total BPRS, the individual BPRS symptom scores with most meaningful changes after drug administration are shown. | | | | | | | | | |

### Summary and conclusions:

A. A single dose of 12 mg 5-MeO-DMT administered via inhalation is well tolerated and induces an astonishing and very significant clinical response in patients formally diagnosed with treatment-resistant major depressive disorder. Such significant clinical response in treatment-resistant patients makes it plausible that a clinical response also occurs in previously untreated patients.
B. The clinical response occurs rapidly within 2 hours after 5-MeO-DMT administration. Such rapid onset is unusual and has not been seen with conventional classes of antidepressants, including tricyclic antidepressants, monoamine oxidase inhibitors, selective serotonin-reuptake inhibitors (SSRIs), serotonin-norepinephrine reuptake inhibitors (SNRls), and others, which generally take 4 to 6 weeks to show their effect.
C. The patients experienced a clinical remission within 2 hours after a single 5-MeO-DMT administration. This is highly superior to any other approved therapy for major depressive disorder, and also to all previously tested psychedelic agents.
D. The clinical response further deepened over the 7-day follow-up period, although 5-MeO-DMT was only given once and is no longer efficaciously present in the body during this time frame (see pharmacokinetic data in Example 3 below). This observation supports the superior clinical profile of 5-MeO-DMT and allows for convenient administration intervals. All conventional classes of antidepressants have been oral medications for chronic daily administration.
E. Besides anti-depressive effects, also endpoints assessing suicidal ideation were positively impacted, supporting the use of 5-MeO-DMT in patients with suicidal ideation, including patients with active suicidal ideation with intent to act, and including patients who are at imminent risk for suicide.
F. Besides anti-depressive effects, also endpoints assessing other psychiatric symptoms (such as somatic concern, anxiety, guilt and tension) were positively impacted, supporting the use of 5-MeO-DMT in patients with other mental diseases.
G. A correlation of the intensity of the acute psychedelic effects after 5-MeO-DMT administration with acute and longer-term anti-depressive treatment effects was observed. This demonstrates the advantage that achieving peak experiences more rapidly, in a larger proportion of patients, and with better reproducibility in an individual patient, will lead to a better therapeutic profile. The observation that peak psychedelic experiences can already occur with low 5-MeO-DMT doses in some patients (such as 6 mg, see example 3 below), while other patients need high doses (such as 18 mg, see example 3 below) supports therapeutic efficacy of a broad range of 5-MeO-DMT dosages, and supports the concept that in patients who have not achieved a peak experience after a first dose of 5-MeO-DMT any subsequent administration should use a dosage amount higher than the previous administration until the patient experiences a peak psychedelic experience.

Each of the aforementioned aspects A) to G) is unexpected, and is medically highly relevant. Aspects A) to G) show that 5-MeO-DMT has a significantly improved efficacy profile compared to approved pharmacological therapies for major depressive disorder and to all previously tested psychedelic agents, when used according to the present invention. Together with the short duration of the acute psychedelic effects and the favorable safety profile, these data show that the technical problem to provide an improved psychoactive therapy in a patient with a major depressive disorder is solved by the present invention. The technical problem is also plausibly solved for persistent depressive disorder, anxiety disorder, posttraumatic stress disorder, body dysmorphic disorder, obsessive-compulsive disorder, eating disorder and psychoactive substance abuse, based on the effects of 5-MeO-DMT on specific psychiatric symptoms observed in this study.

### Example 3 (Reference Example)

### Clinical trial of 5-MeO-DMT administered via inhalation to healthy volunteers

### Study design:

A clinical trial was performed in which 5-MeO-DMT free base (purity not less than 99%) at different dose levels was administered via inhalation (as described in Example 1) in a single day to healthy volunteers who had prior experiences with the use of synthetic or naturally-occurring psychedelics. The trial was reviewed and approved by the relevant national competent authority and local medical ethics committee. The trial consisted of two parts: Part A, where single doses of 5-MeO-DMT dosage amounts of 2 mg, 6 mg, 12 mg and 18 mg were administered; and Part B, where an initial 5-MeO-DMT dosage amount of 6 mg was administered, and then, unless the participant had already experienced a peak psychedelic experience based on the PPEQ or the supervising physician had decided that further dose increases were inappropriate based on observed side effects, further subsequent dosage amounts of 12 mg and 18 mg were administered with about 3 hours in between each administration. Participants in Part A only received one dose and were not allowed to receive higher doses in later parts of Part A and were not allowed to participate in Part B. Participants were closely monitored for 3.5 hours after the last dose, with additional follow-up visits 1 day and 7 days after dosing. Various safety related measures (e.g., adverse event reporting, safety laboratory analyses, vital signs, ECG, CADSS, PVT, DSST, Prospective Memory Task (PMT)) as well as measures of the intensity of the psychedelic effects (MEQ30, ASC, PPEQ, subjective description of the psychedelic experience) were recorded. Pharmacokinetic analysis of 5-MeO-DMT and bufotenine plasma levels was performed at 1 hour and 3 hours after drug administration in Part A, and at 3 hours after drug administration in Part B.

### Participant characteristics:

18 participants were recruited into Part A of the study and 4 participants were recruited into Part B. 9 Participants were female and 13 participants were male. Median age was 29 years (range: 19 to 42 years). In Part A, 4 participants received 2 mg, 6 participants received 6 mg, 4 participants received 12 mg and 4 participants received 18 mg of 5-MeO-DMT. In Part B, 1 participant received 6 mg, 2 participants received 6 mg and then 12 mg and one participant received 6 mg, 12 mg and then 18 mg of 5-MeO-DMT.

### Results:

All participants completed all planned visit days. In Part A, the inhalation procedure was adequately performed by 14 of the 18 participants, while 2 participants performed 2 inhalation sequences to inhale the total inhalation volume, and 2 participants inhaled only about % of the total inhalation volume. In Part B, the inhalation procedure was adequately performed by 3 of the 4 participants, while 1 participant performed an adequate inhalation at the first dose level but inhaled only about % of the total inhalation at the second dose level. No inhalation-related adverse events were reported.

The first psychedelic symptoms occurred within a few seconds after the inhalation in all participants. The duration of the psychedelic experience as judged by an external observer was between 7 minutes and 30 minutes, apparently with no dose-related trends.

Except for a temporary, not clinically relevant increase in heart rate and blood pressure shortly after administration of 5-MeO-DMT, no noteworthy changes in vital parameters occurred. The evaluation of ECG, safety laboratory analyses, CADSS and cognitive tests (PVT, DSST, PMT) was unremarkable.

The reported adverse events were all mild (except for one moderate event) and all resolved spontaneously by the end of the study (Example 3, Table 1 for Part A; Example 3, Table 2 for Part B). While the overall tolerability profile of 5-MeO-DMT was very good, it was observed that the 18 mg dose group in Part A had the highest number of adverse events, and that this was the only dose group where hallucinations and flashbacks of the experience were reported at the day 1 and day 7 timepoints, respectively. In Part B, only few adverse events were reported despite the up to three administrations of 5-MeO-DMT in a single day, and in fact no adverse events were reported for the 18 mg dose (that followed a 6 mg and a 12 mg dose).

### Example 3, Table 1. Adverse events reported in Part A of the study

| **Dose (number of volunteers)** | | **Administration day** | **Day 1** | **Day 7** |
|---|---|---|---|---|
| **2 mg (n=4)** | | | | |
| | Nausea | 2 | | |
| | Tachycardia | 1 | | |
| | Vision blurred | 1 | | |

| **6 mg (n=6)** | | | | |
|---|---|---|---|---|
| | Anxiety | 1 | | |
| | Clumsiness | | 1 | |
| | Feeling hot | | 1 | |
| | Headache | 1 | 1 | |
| | Nausea | 1 | | |

| **12 mg (n=4)** | | | | |
|---|---|---|---|---|
| | Anxiety | 1 | | |
| | Heart rate increased | 1¹ | | |

| **18 mg (n=4)** | | | | |
|---|---|---|---|---|
| | Nausea | 1 | | |
| | Headache | 1 | | |
| | Hyperacusis | | 1 | |
| | Mental fatigue | | 1 | |
| | Flashback | | | 1 |
| | Hallucination | | 1 | |
| | Abnormal dreams | | 1 | |
| | Insomnia | | 1 | |
| | Fatigue | | 1 | |

Shown are possibly related and probably related adverse events. ¹This event was moderate, all other events were mild in severity.

### Example 3, Table 2. Adverse events reported in Part B of the study.

| **Dose (number of volunteers)** | | **Administration day** | **Day 1** | **Day 7** |
|---|---|---|---|---|
| **6 mg (n=4)** | | | | |
| | Nausea | 1 | | |

| **12 mg (n=3), after 6 mg** | | | | |
|---|---|---|---|---|
| | Headache | 1 | | |
| | Nausea | 1 | | |
| | Fatigue | 1 | | |
| | Head discomfort | 1 | | |

| **18 mg (n=1), after 12 mg and 6 mg** | | | | |
|---|---|---|---|---|
| | *None reported* | | | |

Shown are possibly related and probably related adverse events. All events were mild in severity.

With regard to the intensity of the psychedelic experience as measured by the PPEQ in Part A, a dose-related trend for the individual PPEQ questions, for the PPEQ Total Score, and for the fraction of patients who achieved a peak psychedelic experience was observed for the 2 mg, 6 mg and 12 mg dose levels. For the 18 mg dose level, however, this dose-related trend only continued for the Loss of Control question (Example 3, Table 3). Based on the subjective descriptions by the participants, it was found that the psychedelic experience for the 18 mg dose level was associated with a very quick onset but only few memories of the altered state of consciousness in some participants, which may explain this observation (in line with the so-called "white-out" phenomenon). In Part B, where participants started with a 6 mg dosage amount and received further subsequent dosage amounts of 12 mg and 18 mg, if they had not achieved a peak psychedelic experience at the lower dose, it was observed that the intensity of the psychedelic experience increased with the increasing dosage amounts in all participants. Further, all participants were able to achieve a peak psychedelic experience at their maximum individual dose level, which was 6 mg for 1 participant, 12 mg for 2 participants and 18 mg for 1 participant. At this maximum individual dose level, the scores for the individual PPEQ questions and for the PPEQ Total Score were higher than in all dose groups of Part A (Example 3, Table 3). No predictors for the maximum individual dose level that was required to achieve a peak psychedelic experience could be identified.

### Example 3, Table 3. Intensity of the psychedelic effects measured with the PPEQ in Part A and Part B

| | | **Part A 2 mg (n=4)** | **Part A 6 mg (n=6)** | **Part A 12 mg (n=4)** | **Part A 18 mg (n=4)** | **Part B max. dose¹ (n=4)** |
|---|---|---|---|---|---|---|
| **PPEQ** | | | | | | |
| | **Intensity (0-100)** | 16 | 53 | 78 | 62 | 97 |
| | **Loss of Control (0-100)** | 4 | 37 | 69 | 80 | 94 |
| | **Profoundness (0-100)** | 7 | 42 | 51 | 36 | 78 |
| | **PPEQ Total Score (0-100)** | 9.0 | 43.8 | 65.5 | 59.2 | 89.3 |
| | **PPE achieved²** | 0/4 | 1/6 | 2/4 | 1/4 | 4/4 |

Shown are average results for all participants in the respective group. In Part A, participants received only 1 dose. In Part B, one participant received 6 mg and then had a PPE, two participants received 6 mg and then 12 mg and then had a PPE and one participant received 6 mg, then 12 mg and then 18 mg and then had a PPE. PPEQ, Peak Psychedelic Experience Questionnaire; PPE, Peak Psychedelic experience. ¹Shown are data for the highest dose for each participant. ²Shown is the fraction of participants who achieved a PPEQ Total Score of at least 75.

With regard to plasma levels of 5-MeO-DMT, it was found that for any dose in Part A, plasma levels were already very low at the 1-hour timepoint, and that plasma levels in Part A and Part B reached the lower level of quantification (LLOQ) of the 5-MeO-DMT assay (0.014 ng/ml) at the 3-hour time point (Example 3, Table 4). With regard to the plasma levels of the 5-MeO-DMT metabolite bufotenine, minimal amounts below the LLOQ of the bufotenine assay (0.016 ng/ml) were seen in three participants at the 1-hour time point, but bufotenine was undetectable in all other samples.

### Example 3, Table 4. 5-MeO-DMT plasma levels at 1 hour and 3 hours after drug administration

| | **Part A 2 mg (n=4)** | **Part A 6 mg (n=6)** | **Part A 12 mg (n=4)** | **Part A 18 mg (n=4)** | **Part B 6 mg (n=4)** | **Part B 6 mg, 12 mg (n=3)** | **Part B 6 mg, 12 mg, 18 mg (n=1)** |
|---|---|---|---|---|---|---|---|
| **1 hour** | 0.38 (0.03-0.69) | 0.33 (0.09-0.64) | 0.19 (0.13-0.29) | 0.65 (0.15-2.42) | - | - | |
| **3 hours** | 0.03 (0.01-0.04) | 0.02 (0.00-0.06) | 0.01 (0.01-0.01) | 0.04 (0.01-0.24) | 0.01 (0.00-0.09) | 0.03 (0.01-0.05) | 0.05 (0.05-0.05) |

Shown is the group median (range minimum to maximum) of 5-MeO-DMT plasma levels in ng/ml.

### Summary and Conclusions:

A. The tested 5-MeO-DMT dose levels of 2 mg, 6 mg and 12 mg in Part A and the individual dose-escalation scheme in Part B with up to three administrations of 5-MeO-DMT in a single day were well-tolerated in all participants and only few, mainly mild and only short-lasting adverse events occurred.
B. In Part A, initial measurable psychedelic experiences occurred at the 2 mg dose level. More significant psychedelic experiences and a first peak psychedelic experience occurred at the 6 mg dose level. Even stronger psychedelic experiences occurred at the 12 mg dose level, but still only 2/4 participants achieved a peak psychedelic experience. Administering an even higher dose of 18 mg to an unselected group of participants did not show a further increase in psychedelic experience scores and rates of peak psychedelic experiences, which is possibly related to a "white-out" phenomenon in some patients at this high dose level. Also, adverse event rates when given to an unselected group were highest in the 18 mg group of Part A.
C. The individual up-titration regimen as applied in Part B was superior to all individual dose levels in Part A with higher average psychedelic experience ratings, and 4/4 participants achieving a peak psychedelic experience at the individual highest dose. The one participant who was dosed with up to 18 mg in Part B did not experience any adverse events, again supporting that an individual up-titration has a superior therapeutic profile and avoids unnecessary high doses.
D. Plasma levels of 5-MeO-DMT are already very low one hour after administration, and barely measurable at 3 hours. Plasma levels of the 5-MeO-DMT metabolite bufotenine are barely detectable at the 1-hour time point, and undetectable at 3 hours. This pharmacokinetic data aligns well with the short duration of psychedelic effects after administration of 5-MeO-DMT.

In conclusion, the studied dosage amounts between 2 mg and 18 mg administered via inhalation were able to induce psychedelic experiences, and dosage amounts of 6 mg, 12 mg and 18 mg of 5-MeO-DMT administered via inhalation were able to induce peak psychedelic experiences in healthy participants. Because Example 2 makes it plausible that peak psychedelic experiences are associated with a therapeutic benefit in patients diagnosed with major depressive disorder and other mental diseases, it can be predicted that dosage amounts between 4 mg and 20 mg, in particular between 6 mg and 18 mg, of 5-MeO-DMT administered via inhalation are therapeutically effective amounts. An individualized up-titration regimen, as applied in Part B, provides a higher chance to achieve peak psychedelic experiences along with superior tolerability, and therefore provides a superior therapeutic profile compared with administration of a single fixed dose.

### Example 4 (Reference Example)

### The 30-item Revised Mystical Experience Questionnaire (MEQ30) (Barrett FS, J Psychopharmacol. 2015 Nov;29(11):1182-90)

The following questionnaire is applied in situations where the acute psychedelic experience after treatment with 5-MeO-DMT needs to be quantified. At the end of relevant treatment sessions, the patient is handed a paper form and asked to rate the degree to which at any time during that session he experienced any of the phenomena listed below using the scale listed below. The MEQ30 is then computed and the occurrence of a peak psychedelic experience is then evaluated as described under scoring instructions.
Scale:
   0 - none; not at all
   1 - so slight cannot decide
   2 - slight
   3 - moderate
   4 - strong (equivalent in degree to any other strong experience)
   5 - extreme (more than any other time in my life and stronger than 4)
Phenomena:
   1. Loss of usual sense of time.
   2. Experience of amazement.
   3. Sense that the experience cannot be described adequately in words.
   4. Gain of insightful knowledge experienced at an intuitive level.
   5. Feeling that eternity or infinity was experienced.
   6. Experience of oneness or unity with objects and/or persons perceived in your surroundings.
   7. Loss of usual sense of space.
   8. Feelings of tenderness and gentleness.
   9. Certainty of encounter with ultimate reality (in the sense of being able to "know" and "see" what is really real at some point during your experience.
   10. Feeling not to be able to do justice to the experience by describing it in words.
   11. Loss of usual awareness of where one was.
   12. Feelings of peace and tranquility.
   13. Sense of being "outside of" time, beyond past and future.
   14. Freedom from the limitations of the personal self and feeling a unity or bond with what was felt to be greater than the personal self.
   15. Sense of being at a spiritual height.
   16. Experience of pure being and pure awareness (beyond the world of sense impressions).
   17. Experience of ecstasy.
   18. Experience of the insight that "all is One".
   19. Being in a realm with no space boundaries.
   20. Experience of oneness in relation to an "inner world" within.
   21. Sense of reverence.
   22. Experience of timelessness.
   23. Being convinced when looking back at the experience, that ultimate reality was encountered (i.e., that the patient or proband "knew" and "saw" what was really real).
   24. Feeling that something profoundly sacred and holy was experienced.
   25. Awareness of the life or living presence in all things.
   26. Experience of the fusion of the personal self into a larger whole.
   27. Sense of awe or awesomeness.
   28. Experience of unity with ultimate reality.
   29. Feeling that it would be difficult to communicate the own experience to others who have not had similar experiences.
   30. Feelings of joy.

### Scoring Instructions:

The score for each individual subscale is computed by calculating the average score (from the scale of 0 to 5) for the phenomena belonging to the subscale as listed below.

| Subscale | Phenomena belonging to subscale | Maximum possible score |
|---|---|---|
| Mystical | 4, 5, 6, 9, 14, 15, 16, 18, 20, 21, 23, 24, 25, 26, 28 | 75 |
| Positive mood | 2, 8, 12, 17, 27, 30 | 30 |
| Transcendence of time and space | 1, 7, 11, 13, 19, 22 | 30 |
| Ineffability | 3, 10, 29 | 15 |

The MEQ30-total score is computed by taking the average response to all phenomena. Patients or probands with a score ≥60% of the maximum possible score on each of the four subscales of the MEQ30 are considered to have experienced a "peak psychedelic experience".

### Example 5 (Reference Example)

### Peak Psychedelic Experience Questionnaire (PPEQ)

The Peak Psychedelic Experience Questionnaire (PPEQ) has been developed by the inventor as an improved alternative to the oceanic boundlessness dimension of the ASC and the MEQ30 to allow a simpler and quicker assessment of the intensity of a psychedelic experience. The PPEQ is comprised of three questions, all to be scored from 0 to 100 by marking a Visual Analogue Scale between 0 and 100 mm:
1. How intense was the experience?
2. To what extent did you lose control?
3. How profound (i.e., deep and significant) was the experience?

The PPEQTotal Score is the average of the scores of the three questions. A "peak psychedelic experience" is identified through achievement of a PPEQTotal Score of at least 75. A statistically significant correlation between the PPEQ Total Score and the oceanic boundlessness dimension of the ASC, respectively the MEQ30 Total Score, has been observed in the data set described in Example 3.

### Example 6

### Efficacy of 5-MeO-DMT in major depressive disorder - Clinical trial prophetic example

The purpose of this study is to assess the efficacy of 5-MeO-DMT compared with placebo in improving symptoms of major depressive disorder. This first study will be performed in patients with treatment-resistant major depressive disorder, but this is not to be understood that 5-MeO-DMT does not have efficacy and cannot be used in untreated patients with major depressive disorder. The study consists of a screening phase, a randomized, double-blind, placebo-controlled treatment phase, an evaluation phase, and a follow-up phase with optional open-label re-treatment.

Screening Phase: Patients will be screened by a psychiatrist for eligibility for the study based on a comprehensive set of inclusion and exclusion criteria. Patients will also be informed in detail about the study procedures, and effects and potential side effects of the medication and other potential risks.

Patients will confirm their willingness to participate in the study by signing a consent form. Key inclusion criteria for the study are participant 1) must meet Diagnostic and Statistical Manual of Mental Disorders - Fifth Edition (DSM-5) diagnostic criteria for Major Depressive Disorder, without psychotic features, and 2) must have had an inadequate response to at least 2 antidepressants, at least one of which is in the current episode of depression. Key exclusion criteria are 1) patient or immediate family member with current or prior DSM-5 diagnosis of a psychotic disorder, 2) patients with a history of substance abuse within the prior one year, 3) any previous (recreational) use of 5-MeO-DMT, 4) prior participation in any clinical study with any other hallucinogen, 5) known allergies or hypersensitivity or any other contraindication to 5-MeO-DMT or any of the excipients used in the study drug formulation, 6) current treatment with a monoamine oxidase inhibitor such as isocarboxazid, phenelzine, selegiline or tranylcypromine, and 7) positive pregnancy test, lack of appropriate contraception.

Treatment Phase: The study is a double-blind, randomized, placebo-controlled study comparing the administration of 5-MeO-DMT with the administration of placebo. Both compounds are to be provided by intramuscular injection. The 5-MeO-DMT will be provided in the form of its hydrochloride salt and a formulation for intramuscular injection. It is understood that the dosage amounts for 5-MeO-DMT mentioned below relate to the weight amount of the free base and the dosage amounts for the hydrochloride salt of 5-MeO-DMT can be calculated assuming that equimolar amounts are used. Patients will be admitted to the study site in the morning. First, the patient's eligibility to the study will be re-confirmed by the responsible study physician, who is blinded for the assigned treatment group. Second, baseline assessments for the primary endpoint (Montgomery Asberg Depression Rating Scale (MADRS) total score) and all relevant secondary endpoints will be performed by a trained rater who is blinded for the assigned treatment group. Third, the patient will be informed about the application via intramuscular injection. Fourth, the first dose of the study drug (5-MeO-DMT or placebo) will be administered through intramuscular injection. The initial dose to be applied in this study is 2 mg of 5-MeO-DMT or placebo. After the injection, the patient will lie down in a comfortable position and safe environment (e.g. on a mattress on the floor) and will be supervised by the study physician and a second trained observer. The onset of psychedelic symptoms is expected to occur within a few minutes after intramuscular administration. The normal duration of acute psychedelic symptoms after intramuscular administration of the 5-MeO-DMT is about 45 to 60 minutes. After the subjective symptoms have subsided, the intensity of the subjective experience will be assessed by evaluating responses to the 30-item revised Mystical Experience Questionnaire (MEQ30) and the Peak Psychedelic Experience Questionnaire (PPEQ). In case a peak psychedelic experience (defined as at least 60% of the maximum possible score in each of the 4 subscales of the MEQ30 or at least a PPEQ Total Score of 75) occurred, the patient can be discharged, but at the earliest two hours after the last dose. If no peak psychedelic experience occurred, a higher dose of 5-MeO-DMT or placebo, now 5 mg, will be administered at 3 hours after the first dose according to the same procedure as for the first dose. A third dose, now 8 mg, can be given after another 3-hour interval, if no peak psychedelic experience has been achieved and no intolerable side effects have occurred with any of the prior doses.

Evaluation Phase: The patient will have follow-up visits at days 7, 14, and 28 after the last dose of study medication. Efficacy and safety evaluations will be performed at each of those visits. The primary endpoint of the study will be the change from baseline to day 7 in the MADRS total score. Key secondary endpoints will be the sustained response in the MADRS total score (50% reduction from baseline in MADRS total score) at day 28 and the change from baseline in the 16-item Quick Inventory of Depressive Symptomatology - Self Report (QIDS-SR16) at day 7 and 28. Further efficacy assessments include achievement of remission, defined as a MADRS total score <=10, the Hamilton Depression Rating scale (HAM-D), the Clinical Global Impression - Severity (CGI-S) and the Patient Global Impression - Severity (PGI-S), and Generalized Anxiety Disorder 7-item Scale (GAD-7). All endpoints will be assessed at each time point by a trained rater who is blinded for the assigned treatment group.

Follow-up phase, and optional open-label retreatment: Patients and physicians will be unblinded regarding the treatment group association after the 28-day visit, but further monthly follow-up visits with efficacy and safety evaluations similar to the evaluations during the blinded phase will be performed. Patients who had achieved at least a 50% reduction from baseline in MADRS total score at the primary endpoint will be eligible for three-monthly re-treatment with 5-MeO-DMT using the highest dose given in the blinded phase, with the first re-treatment on day 28.

Analysis: The treatment will be deemed effective if a significant difference can be detected for the change from baseline to day 7 in the MADRS total score for the active 5-MeO-DMT arm compared with the placebo arm, as defined in the statistical analysis plan of the study. The analysis of secondary endpoints can provide further evidence of clinical efficacy.

### Example 7 (Reference Example)

### Efficacy of 5-MeO-DMT in anxiety disorder - Clinical trial prophetic example

The purpose of this study is to assess the efficacy of 5-MeO-DMT compared with active control in improving symptoms of anxiety disorders. This first study will be performed in patients with treatment-resistant anxiety disorders, but this is not to be understood that 5-MeO-DMT does not have efficacy and cannot be used in untreated patients with anxiety disorders. The study consists of a screening phase, a randomized, active-controlled, treatment phase, an evaluation phase, cross-over of participants into the other study arm and then another active-controlled treatment phase and evaluation phase, and a follow-up phase with optional open-label re-treatment.

Screening Phase: Patients will be screened by a psychiatrist for eligibility for the study based on a comprehensive set of inclusion and exclusion criteria. Patients will also be informed in detail about the study procedures, and effects and potential side effects of the medication and other potential risks. Patients will confirm their willingness to participate in the study by signing a consent form. Key inclusion criteria for the study are participant 1) must meet DSM-5 diagnostic criteria for social anxiety disorder, generalized anxiety disorder, and/or panic disorder and 2) must have failed to achieve remission with at least one adequate prior anxiolytic medication (e.g. selective serotonin reuptake inhibitors) meaning at least 8 weeks at therapeutic dosing, including at least 4 weeks of stable dosing and must have failed to achieve remission with previous cognitive behavioral therapy or must have declined current cognitive behavioral therapy. Key exclusion criteria are 1) patient or immediate family member with current or prior DSM-5 diagnosis of a psychotic disorder, 2) patients with a history of substance abuse within the prior one year, 3) any previous (recreational) use of 5-MeO-DMT, 4) prior participation in any clinical study with any other hallucinogen, 5) known allergies or hypersensitivity or any other contraindication to 5-MeO-DMT or midazolam or any of the excipients used in the study drug formulations, 6) current treatment with a monoamine oxidase inhibitor such as isocarboxazid, phenelzine, selegiline or tranylcypromine, and 7) positive pregnancy test, lack of appropriate contraception.

Treatment Phase: The study is a randomized, active-controlled study comparing the administration of 5-MeO-DMT with the administration of midazolam. 5-MeO-DMT is to be provided via intravenous injection. The 5-MeO-DMT will be provided in the form of its hydrochloride salt and a formulation for intravenous injection. It is understood that the dosage amounts for 5-MeO-DMT mentioned below relate to the weight amount of the free base and the dosage amounts for the hydrochloride salt of 5-MeO-DMT can be calculated assuming that equimolar amounts are used. Midazolam is to be provided via intravenous infusion. Patients will be admitted to the study site in the morning. First, the patient's eligibility to the study will be re-confirmed by the responsible study physician, who is blinded for the assigned treatment group. Second, baseline assessments for the primary endpoint (the Hamilton Anxiety Scale (HAM-A)) and all relevant secondary endpoints will be performed by a trained rater who is blinded for the assigned treatment group. Third, the study drug will be administered, as intravenous injection, at an initial dose of 1 mg, and Midazolam via intravenous infusion over 40 minutes at a dose of 0.045 mg/kg of Midazolam. After study drug administration, the patient will lie down in a comfortable position and safe environment (e.g. on a mattress on the floor) and will be supervised by the study physician and a second trained observer.

The onset of psychedelic symptoms in the 5-MeO-DMT group is expected to occur within a few seconds after administration injection of the 5-MeO-DMT. The normal duration of acute psychedelic symptoms after administration of the 5-MeO-DMT is 5 to 20 minutes. After the subjective symptoms have subsided in both groups, the intensity of the subjective experience will be assessed by evaluating responses to the 30-item revised Mystical Experience Questionnaire (MEQ30) and the Peak Psychedelic Experience Questionnaire (PPEQ). The midazolam group can now be discharged, but at the earliest two hours after the last dose. The 5-MeO-DMT group can also be discharged in case a peak psychedelic experience (defined as at least 60% of the maximum possible score in each of the 4 subscales of the MEQ30 or at least a PPEQ Total Score of 75) has occurred, but at the earliest two hours after the last dose. If no peak psychedelic experience occurred, a higher dose of 5-MeO-DMT, now 2 mg, will be administered at 3 hours after the first dose according to the same procedure as for the first dose. A third dose, now 3 mg, can be given after another 3-hour interval, if no peak psychedelic experience has been achieved and no intolerable side effects have occurred with any of the prior doses.

Evaluation Phase: The patient will have follow-up visits at days 1, 7, 14, and 28 after the last dose of study medication. Efficacy and safety evaluations will be performed at each of those visits. The primary endpoint of the study will be the change from baseline to day 28 in the HAM-A total score. Key secondary endpoints will be the change from baseline to day 1, 7, 14 and 28 in Clinical Global Impression - Improvement (CGI-I) and Patient Global Impression - Improvement (PGI-I). All endpoints will be assessed at each time point by a trained rater who is blinded for the assigned treatment group.

Cross-over and second evaluation phase: Patients will cross over to the other study arm on day 28, and patients will receive treatment of the other study group and evaluation as described above.

Follow-up phase, and optional open-label retreatment: Patients and physicians will be unblinded with regard to the treatment group association after the 56-day visit, but further monthly follow-up visits with efficacy and safety evaluations similar to the evaluations during the initial phase will be performed. Patients who had achieved at least a 50% reduction from baseline in HAM-A total score at the primary endpoint (or after cross-over at day 56) will be eligible for three-monthly re-treatment with 5-MeO-DMT using the highest dose given in the blinded phase.

Analysis: The treatment will be deemed effective if a significant difference can be detected for the change from baseline to day 28 in the HAM-A total score for the active 5-MeO-DMT arm compared with the midazolam arm, as defined in the statistical analysis plan of the study. The analysis of secondary endpoints can provide further evidence of clinical efficacy.

### Example 8 (Reference Example)

### Efficacy of 5-MeO-DMT in anorexia nervosa - Clinical trial prophetic example

The purpose of this study is to assess the efficacy of 5-MeO-DMT in improving symptoms of anorexia nervosa. The study consists of a screening phase, a treatment phase, an evaluation phase, and a follow-up phase with optional re-treatment.

Screening Phase: Patients will be screened by a psychiatrist for eligibility for the study based on a comprehensive set of inclusion and exclusion criteria. Patients will also be informed in detail about the study procedures, and effects and potential side effects of the medication and other potential risks. Patients will confirm their willingness to participate in the study by signing a consent form. Key inclusion criteria for the study are participant 1) must meet DSM-5 diagnostic criteria for anorexia nervosa, 2) must have a body mass index less than 19 kg/m2 and greater than 14 kg/m2 and 3) must have had a duration of disease of at least 5 years. Key exclusion criteria are 1) patient or immediate family member with current or prior DSM-5 diagnosis of a psychotic disorder, 2) patients with a history of substance abuse within the prior one year, 3) any previous (recreational) use of 5-MeO-DMT, 4) prior participation in any clinical study with any other hallucinogen, 5) known allergies or hypersensitivity or any other contraindication to 5-MeO-DMT or any of the excipients used in the study drug formulation, 6) current treatment with a monoamine oxidase inhibitor such as isocarboxazid, phenelzine, selegiline or tranylcypromine, and 7) positive pregnancy test, lack of appropriate contraception.

Treatment Phase: The study is a single arm study. 5-MeO-DMT is to be provided via intramuscular injection. The 5-MeO-DMT will be provided in the form of its hydrochloride salt and a formulation for intramuscular injection. It is understood that the dosage amounts for 5-MeO-DMT mentioned below relate to the weight amount of the free base and the dosage amounts for the hydrochloride salt of 5-MeO-DMT can be calculated assuming that equimolar amounts are used. Patients will be admitted to the study site in the morning of the first treatment day. First, the patient's eligibility to the study will be re-confirmed by the responsible study physician. Second, baseline assessments for the primary endpoint and all relevant secondary endpoints will be performed by a trained rater. Third, the patient will be informed about the application of the study drug via intramuscular injection. Fourth, the first dose of the study drug will be administered under the supervision of the responsible study physician. The initial dose to be applied in this study is 2 mg of 5-MeO-DMT. After the administration, the patient will lie down in a comfortable position and safe environment (e.g. on a mattress on the floor) and will be supervised by the study physician and a second trained observer. The onset of psychedelic symptoms is expected to occur within a few minutes after intramuscular administration. The normal duration of acute psychedelic symptoms after intramuscular administration of the 5-MeO-DMT is about 45 to 60 minutes. After the subjective symptoms have subsided, the intensity of the subjective experience will be assessed by evaluating responses to the 30-item revised Mystical Experience Questionnaire (MEQ30) and the Peak Psychedelic Experience Questionnaire (PPEQ. In case a peak psychedelic experience (defined as at least 60% of the maximum possible score in each of the 4 subscales of the MEQ30 or at least a PPEQ Total Score of 75) occurred, the patient can be discharged, but at the earliest three hours after the last dose. If no peak psychedelic experience occurred, a higher dose of 5-MeO-DMT, now 5 mg, will be administered at 3 hours after the first dose according to the same procedure as for the first dose. A third dose, now 8 mg, can be given after another 3-hour interval, if no peak psychedelic experience has been achieved and no intolerable side effects have occurred with any of the prior doses.

Evaluation Phase: The patient will have follow-up visits at days 7, 14, 28 and 56 after the last dose of study medication. Efficacy and safety evaluations will be performed at each of those visits. The primary endpoint of the study will be the change from baseline to day 56 in BMI of the patient. Key secondary endpoints will be change from baseline to day 56 in Eating Disorder Inventory (EDI) scale and change from baseline to day 56 in the MADRS total score. Further efficacy assessments include the Clinical Global Impression - Severity (CGI-S) and the Patient Global Impression - Severity (PGI-S). All endpoints will be assessed at each time point by a trained rater.

Follow-up phase, and optional retreatment: After the 56-day visit further monthly follow-up visits with efficacy and safety evaluations similar to the evaluations during the initial phase will be performed. Patients who had achieved at least a 1-point increase in their BMI from baseline at the primary endpoint will be eligible for three-monthly re-treatment with 5-MeO-DMT using the same uptitration schedule as for the first treatment, with the first re-treatment on day 56.

Analysis: The treatment will be deemed effective if the average change in BMI at day 56 is significantly greater than 0.5 kg/m². The analysis of secondary endpoints can provide further evidence of clinical efficacy.

### Citation List

Barrett FS, Validation of the revised Mystical Experience Questionnaire in experimental sessions with psilocybin. J Psychopharmacol. 2015; 29(11):1182-90
Bogenschutz MP et al., Psilocybin-assisted treatment for alcohol dependence: a proof-of-concept study. J Psychopharmacol. 2015; 29(3):289-99
Carhart-Harris et al., Psilocybin with psychological support for treatment-resistant depression: six-month follow-up. Psychopharmacology (Berl). 2018; 235(2):399-408
Carhart-Harris RL et al., Neural correlates of the LSD experience revealed by multimodal neuroimaging. Proc Natl Acad Sci USA. 2016; 113(17):4853-8
Carhart-Harris RL et al., Psilocybin for treatment-resistant depression: fMRI-measured brain mechanisms. Sci Rep. 2017;7(1):13187
Carhart-Harris RL et al., Psilocybin with psychological support for treatment-resistant depression: an open-label feasibility study. Lancet Psychiatry. 2016; 3(7):619-27
Davis AK et al., The epidemiology of 5-Methoxy-N,N-Dimethyltryptamine (5-MeO-DMT) use: Benefits, consequences, patterns of use, subjective effects, and reasons for consumption. J Psychopharmacol. 2018 Jul;32(7):779-792
Garcia-Romeu A, Psilocybin-occasioned mystical experiences in the treatment of tobacco addiction. Curr Drug Abuse Rev. 2014;7(3):157-64
Gasser P et al., Safety and efficacy of lysergic acid diethylamide-assisted psychotherapy for anxiety associated with life-threatening diseases. J Nerv Ment Dis. 2014; 202(7):513-20
Griffiths RR et al., Psilocybin produces substantial and sustained decreases in depression and anxiety in patients with life-threatening cancer: A randomized double-blind trial. J Psychopharmacol. 2016; 30(12):1181-1197)
Jensen SE, A treatment program for alcoholics in a mental hospital. QJ Stud Alcohol. 1962; 23:315-20
Johnson MW et al., Long-term follow-up of psilocybin-facilitated smoking cessation. Am J Drug Alcohol Abuse. 2017; 43(1):55-60
Johnson MW et al., Pilot study of the 5-HT2AR agonist psilocybin in the treatment of tobacco addiction. J Psychopharmacol. 2014; 28(11):983-92
MacLean JR et al., The use of LSD-25 in the treatment of alcoholism and other psychiatric problems. Q J Stud Alcohol. 1961; 22:34-45
Moreno et al., Safety, tolerability, and efficacy of psilocybin in 9 patients with obsessive-compulsive disorder. J Clin Psychiatry. 2006; 67(11):1735-40
Pahnke WN et al., LSD-assisted psychotherapy with terminal cancer patients. In: Psychedelic drugs. Proceedings of a Hahnemann Medical College and Hospital Symposium. Editors: Richard E. Hicks, Paul Jay Fink, Van Buren O. Hammett. Grune & Stratton, New York/London 1969, pp.33-42
Palhano-Fontes F et al., Rapid antidepressant effects of the psychedelic ayahuasca in treatment-resistant depression: a randomised placebo-controlled trial. bioRxiv 103531. 2017; doi: https://doi.org/10.1101/103531
Palhano-Fontes F et al., The psychedelic state induced by ayahuasca modulates the activity and connectivity of the default mode network. PLoS One. 2015;10(2):e0118143
Roseman Let al., Quality of Acute Psychedelic Experience Predicts Therapeutic Efficacy of Psilocybin for Treatment-Resistant Depression. Front Pharmacol. 2018; 8:974
Ross S et al., Rapid and sustained symptom reduction following psilocybin treatment for anxiety and depression in patients with life-threatening cancer: a randomized controlled trial. J Psychopharmacol. 2016; 30(12):1165-1180
Unger SM, Mescaline, LSD, Psilocybin, and Personality Change. Psychiatry. 1963; 26:111-25 https://erowid.org/chemicals/5meo_dmt/5meo_dmt_dose.shtml, accessed March 1, 2018. https://erowid.org/chemicals/5meo dmt/5meo dmt_effects.shtml, accessed March 1, 2018

## Claims

1. 5-Methoxy-N,N-dimethyltryptamine (5-MeO-DMT) or a pharmaceutically acceptable salt thereof for use in treating major depressive disorder in a patient who is diagnosed with major depressive disorder by a licensec professional in accordance with accepted medical practice, wherein the 5-MeO-DMT is administered via the intravenous, intramuscular or subcutaneous route.

2. 5-MeO-DMT or a pharmaceutically acceptable salt thereof for use as in claim 1, wherein the disorder is diagnosed in accordance with the Diagnostic and Statistical Manual of Mental Disorders - Fifth Edition (DSM-5) published by the American Psychiatric Association.

3. 5-MeO-DMT or a pharmaceutically acceptable salt thereof for use as in claims 1 or 2, wherein the patient suffers from moderate or severe major depressive disorder as indicated by a Montgomery-Åsberg Depression Rating Scale (MADRS) score of 20 or more or by a 17-item Hamilton Depression Rating Scale (HAM-D) score of 17 or more.

4. 5-MeO-DMT or a pharmaceutically acceptable salt thereof for use as in claim 3, wherein the patient suffers from severe major depressive disorder as indicated by a MADRS score of 35 or more or by a HAM-D score of 25 or more.

5. 5-MeO-DMT or a pharmaceutically acceptable salt thereof for use as in claims 1 to 4, wherein the patient is diagnosed with a treatment-resistant form of major depressive disorder.

## Patentansprüche

1. 5-Methoxy-N,N-dimethyltryptamin (5-MeO-DMT) oder ein pharmazeutisch annehmbares Salz davon zur Verwendung bei der Behandlung von schwerer depressiver Störung bei einem Patienten, bei dem schwere depressive Störung von einer zugelassenen Fachkraft in Übereinstimmung mit der anerkannten medizinischen Praxis diagnostiziert wurde, wobei das 5-MeO-DMT intravenös, intramuskulär oder subkutan verabreicht wird.

2. 5-MeO-DMT oder ein pharmazeutisch akzeptables Salz davon zur Verwendung gemäß Anspruch 1, wobei die Störung gemäß dem von der American Psychiatric Association veröffentlichten Diagnostic and Statistical Manual of Mental Disorders - Fifth Edition (DSM-5) diagnostiziert wird.

3. 5-MeO-DMT oder ein pharmazeutisch annehmbares Salz davon zur Verwendung gemäß Anspruch 1 oder 2, wobei der Patient an einer mittelschweren oder schweren starken depressiven Störung leidet, wie sie durch einen Wert auf der Montgomery-Äsberg Depression Rating Scale (MADRS) von 20 oder mehr oder durch einen Wert auf der 17-teiligen Hamilton Depression Rating Scale (HAM-D) von 17 oder mehr angezeigt wird.

4. 5-MeO-DMT oder ein pharmazeutisch annehmbares Salz davon zur Verwendung gemäß Anspruch 3, wobei der Patient an einer schweren starken depressiven Störung leidet, die durch einen MADRS-Score von 35 oder mehr oder durch einen HAM-D-Score von 25 oder mehr angezeigt wird.

5. 5-MeO-DMT oder ein pharmazeutisch annehmbares Salz davon zur Verwendung gemäß den Ansprüchen 1 bis 4, wobei bei dem Patienten eine behandlungsresistente Form einer schweren depressiven Störung diagnostiziert wird.

## Revendications

1. 5-Méthoxy-N,N-diméthyltryptamine (5-MeO-DMT) ou un de ses sels pharmaceutiquement acceptables destiné à être utilisé dans le traitement du trouble dépressif majeur chez un patient chez qui un professionnel agréé a diagnostiqué trouble dépressif majeur, conformément aux pratiques médicales reconnues, le 5-MeO-DMT étant administré par voie intraveineuse, intramusculaire ou sous-cutanée.

2. 5-MeO-DMT ou un de ses sels pharmaceutiquement acceptables pour l'utilisation selon la revendication 1, dans lequel le trouble est diagnostiqué conformément au Manuel diagnostique et statistique des troubles mentaux - Cinquième édition (DSM-5) publié par l'American Psychiatric Association.

3. 5-MeO-DMT ou un de ses sels pharmaceutiquement acceptables pour une utilisation telle que décrite dans les revendications 1 ou 2, dans laquelle le patient souffre d'un trouble dépressif majeur modéré ou sévère tel qu'indiqué par un score de 20 ou plus sur l'échelle d'évaluation de la dépression de Montgomery- Åsberg (MADRS) ou par un score de 17 ou plus sur l'échelle d'évaluation de la dépression de Hamilton (HAM-D).

4. 5-MeO-DMT ou un de ses sels pharmaceutiquement acceptables pour l'utilisation selon la revendication 3, dans lequel le patient souffre d'un trouble dépressif majeur sévère tel qu'indiqué par un score MADRS de 35 ou plus ou par un score HAM-D de 25 ou plus.

5. 5-MeO-DMT ou un de ses sels pharmaceutiquement acceptables pour une utilisation telle que décrite dans les revendications 1 à 4, dans laquelle le patient est diagnostiqué comme souffrant d'une forme résistante au traitement du trouble dépressif majeur.
